(19) Europäisches Patentamt
European Patent Office
Office européen des brevets

(11) Veröffentlichungsnummer: **0 161 599**
**A2**

# (12) EUROPÄISCHE PATENTANMELDUNG

(21) Anmeldenummer: **85105477.5**

(22) Anmeldetag: **06.05.85**

(51) Int. Cl.[4]: **C 07 D 223/16**, A 61 K 31/55
// C07C103/30, C07C59/64

(30) Priorität: **17.05.84 DE 3418271**

(43) Veröffentlichungstag der Anmeldung: **21.11.85**
**Patentblatt 85/47**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Reiffen, Manfred, Dr. Dipl.-Chem., Matthias-Erzberger-Strasse 40, D-7950 Biberach 1 (DE)**
Erfinder: **Noll, Klaus, Dr. Dipl.-Chem., Im Schönblick 3, D-7951 Warthausen (DE)**
Erfinder: **Heider, Joachim, Dr. Dipl.-Chem., Am Hang 3, D-7951 Warthausen (DE)**
Erfinder: **Austel, Volkhard, Dr. Dipl.-Chem., Kapellenweg 7, D-7950 Biberach 1 (DE)**
Erfinder: **Hauel, Norbert, Dr. Dipl.-Chem., Händelstrasse 12, D-7950 Biberach 1 (DE)**
Erfinder: **Kobinger, Walter, Prof. Dr., Belghofergasse 27, A-1121 Wien (AT)**
Erfinder: **Lillie, Christian, Dr., Hansl-Niese-Weg 12, A-1130 Wien (AT)**

## (54) Neue Benzazepinderivate, diese Verbindungen enthaltende Arzneimittel und Verfahren zu ihrer Herstellung.

(57) Die vorliegende Erfindung betrifft neue Benzazepinderivate der allgemeinen Formel

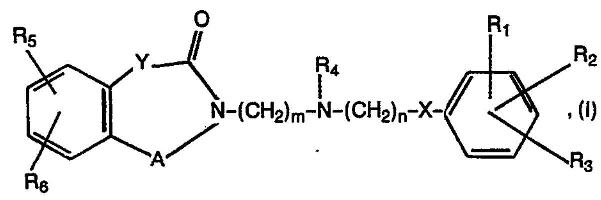
, (I)

in der
A eine $-CH_2CH_2-$ oder $-CH=CH-$Gruppe,
$R_1$ ein Wasserstoff-, Chlor- oder Bromatom, eine Alkyl-, Amino-, Alkylamino-, Dialkylamino-, Acylamino-, Hydroxy-, Alkoxy- oder Phenylalkoxygruppe,
$R_2$ ein Wasserstoff-, Chlor- oder Bromatom, eine Hydroxy-, Alkyl-, Alkoxy- oder Phenylalkoxygruppe oder
$R_1$ und $R_2$ zusammen eine Alkylendioxigruppe,
$R_3$ Wasserstoff-, Chlor- oder Bromatome oder eine Alkoxygruppe,
$R_4$ ein Wasserstoffatom, eine Benzyl-, Alkyl- oder Alkenylgruppe,
$R_5$ ein Wasserstoff- oder Halogenatom, eine Alkyl- oder Alkoxygruppe,
$R_6$ ein Wasserstoffatom, eine Alkyl- oder Alkoxygruppe oder zusammen mit $R_5$ eine Alkylendioxigruppe,
X eine gegebenenfalls durch eine Benzyl- oder Alkylgruppe substituierte Iminogruppe, ein Sauerstoff- oder Schwefelatom, eine Sulfinyl- oder Sulfonylgruppe,
Y eine gegebenenfalls durch eine Benzyl- oder Alkylgruppe substituierte Iminogruppe, eine Methylen- oder Carbonylgruppe,
n die Zahlen 2, 3 oder 4 und
m die Zahlen 2, 3 oder 4 bedeuten, und deren Säureadditionssalze mit anorganischen oder organischen Säuren, welche wertvolle pharmakologische Eigenschaften aufweisen, insbesondere eine herzfrequenzsenkende Wirkung und eine herabsetzende Wirkung auf den $O_2$-Bedarf des Herzens.

Die neuen Verbindungen lassen sich nach an sich bekannten Methoden herstellen.

EP 0 161 599 A2

DR. KARL THOMAE GMBH
D-7950 Biberach 1

0161599
Case 5/909
Dr. Fl/Kp

# Neue Benzazepinderivate, diese Verbindungen enthaltende Arzneimittel und Verfahren zu ihrer Herstellung

Gegenstand der vorliegenden Erfindung sind neue Benzazepinderivate der allgemeinen Formel

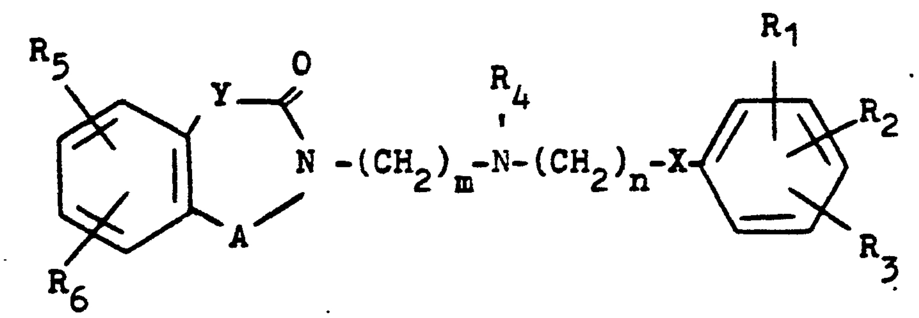

,(I)

deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Die neuen Verbindungen weisen wertvolle pharmakologische Eigenschaften auf, insbesondere eine langanhaltende herzfrequenzsenkende Wirkung und eine herabsetzende Wirkung auf den $O_2$-Bedarf des Herzens.

In der obigen allgemeinen Formel I bedeutet

A eine $-CH_2CH_2-$ oder $-CH=CH-$Gruppe,

$R_1$ ein Wasserstoff-, Chlor- oder Bromatom, eine Alkyl-, Amino-, Alkylamino-, Dialkylamino-, Acylamino-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkyl-, Alkoxy- oder Phenylalkoxygruppe, wobei der Alkylteil jeweils 1 bis 3 Kohlenstoffatome enthalten kann, oder

$R_1$ und $R_2$ zusammen eine Alkylendioxigruppe mit 1 oder 2 Kohlenstoffatomen,

$R_3$ ein Wasserstoff-, Chlor- oder Bromatom oder eine Alkoxygruppe mit 1 bis 3 Kohlenstoffatomen,

$R_4$ ein Wasserstoffatom, eine Benzyl-, Alkyl- oder Alkenylgruppe, wobei der Alkylteil 1 bis 3 Kohlenstoffatome und der Alkenylteil 3 bis 5 Kohlenstoffatome enthalten kann,

$R_5$ ein Wasserstoff- oder Halogenatom, eine Alkyl- oder Alkoxygruppe mit jeweils 1 bis 3 Kohlenstoffatomen,

$R_6$ ein Wasserstoffatom, eine Alkyl- oder Alkoxygruppe mit jeweils 1 bis 3 Kohlenstoffatomen oder

$R_5$ und $R_6$ zusammen eine Alkylendioxigruppe mit 1 oder 2 Kohlenstoffatomen,

X eine gegebenenfalls durch eine Benzylgruppe oder eine Alkylgruppe mit 1 bis 3 Kohlenstoffatomen substituierte Iminogruppe, ein Sauerstoff- oder Schwefelatom, eine Sulfinyl- oder Sulfonylgruppe,

Y eine gegebenenfalls durch eine Benzylgruppe oder eine Alkylgruppe mit 1 bis 3 Kohlenstoffatomen substituierte Iminogruppe, eine Methylen- oder Carbonylgruppe,

n die Zahlen 2, 3 oder 4 und

m die Zahlen 2, 3 oder 4.

Für die bei der Definition der Reste eingangs erwähnten Bedeutungen kommt beispielsweise

für $R_1$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Formylamino-, Acetylamino-, Propionylamino-, Benzyloxy-, 1-Phenylethoxy-, 1-Phenylpropoxy-, 2-Phenylethoxy- oder 3-Phenylpropoxygruppe,

für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,

für $R_3$ die des Wasserstoff-, Chlor- oder Bromatoms, der Methoxy-, Ethoxy-, n-Propoxy- oder Isopropoxygruppe,

für $R_4$ die des Wasserstoffatoms, der Methyl-, Ethyl-, n-Propyl-, Isopropyl-, Benzyl-, Allyl-, Buten-(2)-yl- oder Penten-(2)-ylgruppe,

für $R_5$ die des Wasserstoff-, Fluor-, Chlor- oder Bromatoms, der Methyl-, Ethyl-, n-Propyl-, Isopropyl-, Methoxy-, Ethoxy-, n-Propoxy- oder Isopropoxygruppe,

für $R_6$ die des Wasserstoffatoms, der Methyl-, Ethyl-, n-Propyl-, Isopropyl-, Methoxy-, Ethoxy-, n-Propoxy- oder Isopropoxygruppe oder

zusammen mit $R_5$ die Methylendioxy- oder Ethylendioxygruppe,

für X die des Sauerstoff- oder Schwefelatoms, der Sulfinyl-, Sulfonyl-, Imino-, Methylimino-, Ethylimino-, n-Propylimino- oder Benzyliminogruppe und

für Y die der Methylen-, Carbonyl-, Imino-, Methylimino-, Ethylimino-, n-Propylimino- oder Benzyliminogruppe in Betracht.

Bevorzugte Verbindungen sind jedoch die Verbindungen der allgemeinen Formel I, in der

A eine $-CH_2CH_2$-Gruppe,

Y eine $-CH_2$-, -CO- oder NH-Gruppe,

X ein Sauerstoff- oder Schwefelatom, eine NH-, $NCH_3$-, SO- oder $SO_2$-Gruppe,

n die Zahlen 2, 3 oder 4,

m die Zahl 3,

$R_1$ ein Wasserstoffatom, eine Methoxy-, Acetylamino-, Amino-, Methylamino- oder Dimethylaminogruppe,

$R_2$ ein Wasserstoff-, Chlor- oder Bromatom, eine Methoxygruppe oder $R_1$ und $R_2$ zusammen eine Methylendioxygruppe,

$R_3$ ein Wasserstoff-, Chlor- oder Bromatom oder eine Methoxygruppe,

$R_4$ ein Wasserstoffatom oder eine Methylgruppe,

$R_5$ und $R_6$ jeweils eine Methoxygruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel I sind jedoch diejenigen, in denen

A die $-CH_2CH_2$-Gruppe,

Y eine $-CH_2$-Gruppe,

X ein Sauerstoffatom, eine Imino-, Methylimino-, Sulfinyl- oder Sulfonylgruppe,

n die Zahlen 2 und 3,

m die Zahl 3,

$R_1$ ein Wasserstoffatom, eine Methoxy- oder Aminogruppe,

$R_2$ ein Wasserstoff-, Chlor- oder Bromatom, eine Methoxygruppe oder $R_1$ und $R_2$ zusammen eine Methylendioxygruppe,

$R_3$ ein Wasserstoff-, Chlor- oder Bromatom,

$R_4$ ein Wasserstoffatom oder eine Methylgruppe,

$R_5$ in 7-Stellung eine Methoxygruppe und

$R_6$ in 8-Stellung eine Methoxygruppe bedeuten, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Umsetzung einer Verbindung der allgemeinen Formel

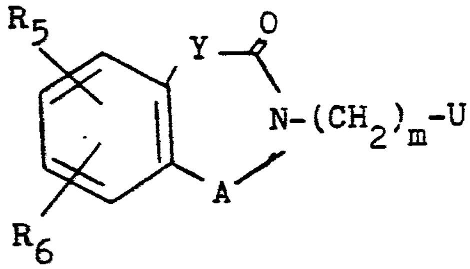

,(II)

mit einer Verbindung der allgemeinen Formel

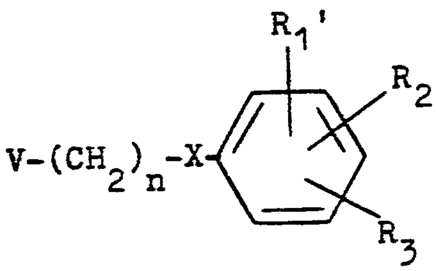

,(III)

in denen

A, $R_2$, $R_3$, $R_5$, $R_6$, X, Y, m und n wie eingangs definiert sind,

$R_1$' eine durch einen Schutzrest geschützte Hydroxy-, Amino- oder Alkylaminogruppe darstellt oder die für $R_1$ eingangs erwähnten Bedeutungen besitzt,

einer der Reste U oder V die $R_4$-NH-Gruppe darstellt, wobei $R_4$ wie eingangs definiert ist, und

der andere der Reste U oder V eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, bedeuten, und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart

einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b) Zur Herstellung von Verbindungen der allgemeinen Formel I, in der $R_4$ eine Alkylgruppe und/oder $R_1$ eine Dialkylaminogruppe darstellt und X wie eingangs definiert ist oder eine durch eine Alkylgruppe mit jeweils 1 bis 3 Kohlenstoffatomen substituierte Iminogruppe darstellt:

Umsetzung einer Verbindung der allgemeinen Formel

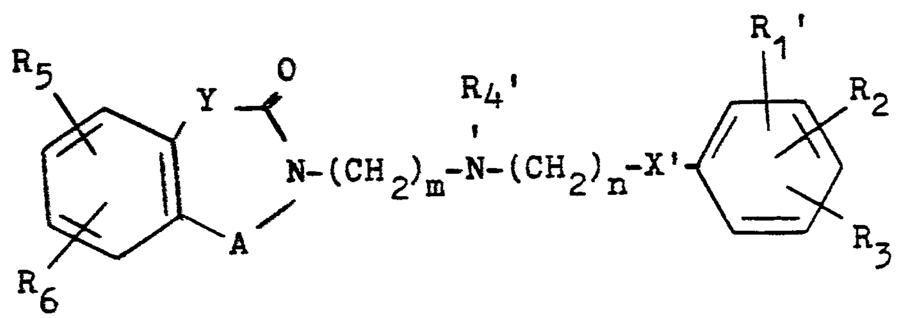

,(IV)

in der
A, $R_2$, $R_3$, $R_5$, $R_6$, Y, m und n wie eingangs definiert sind,

$R_1$' eine durch einen Schutzrest geschützte Hydroxy-, Amino- oder Alkylaminogruppe darstellt oder die für $R_1$ eingangs erwähnten Bedeutungen besitzt,

$R_4$' ein Wasserstoffatom darstellt oder die für $R_4$ eingangs erwähnten Bedeutungen besitzt und

X' eine Iminogruppe darstellt oder die für X eingangs erwähnten Bedeutungen besitzt, wobei jedoch entweder $R_4$' ein Wasserstoffatom oder X' eine Iminogruppe darstellen muß, mit einer Verbindung der Formel

$$R_7 - CHO \qquad ,(V)$$

in der
$R_7$ ein Wasserstoffatom, eine Methyl- oder Äthylgruppe darstellt, in Gegenwart eines Reduktionsmittels.

Die Umsetzung wird zweckmäßigerweise in einem geeigneten Lösungsmittel oder Lösungsmittelgemisch wie Wasser, Wasser/Methanol, Methanol, Ethanol, Ethanol/Essigsäureethylester oder Dioxan in Gegenwart eines Reduktionsmittels, z.B. in Gegenwart von Ameisensäure, von katalytisch angeregtem Wasserstoff oder eines komplexen Metallhydrids, bei Temperaturen zwischen 0 und 100°C, vorzugsweise jedoch bei Temperaturen zwischen 20 und 80°C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6-7 und bei Raumtemperatur durchgeführt.

c) Zur Herstellung von Verbindungen der allgemeinen Formel I, in der X eine Sulfinyl- oder Sulfonylgruppe darstellt:

Oxidation einer Verbindung der allgemeinen Formel

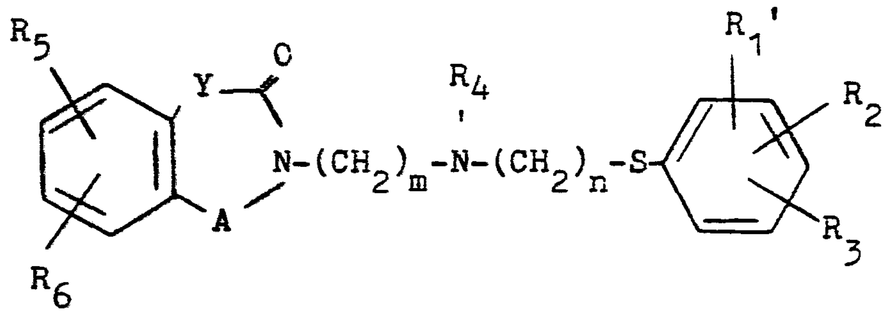

,(VI)

in der
A, $R_2$ bis $R_6$, Y, m und n wie eingangs definiert sind und

$R_1'$ eine durch eine Schutzgruppe geschützte Hydroxy-, Amino- oder Alkylaminogruppe darstellt oder die für $R_1$ eingangs erwähnten Bedeutungen besitzt, und gegebenenfalls anschließende Abspaltung eines verwendeten Schutzrestes.

Die Oxidation wird in einem Lösungsmittel wie Eisessig, Chloroform oder Aceton in Gegenwart eines Oxidationsmittels wie Wasserstoffperoxid, Kaliumpermanganat, m-Chlorperbenzoesäure oder Kaliumpersulfat bei Temperaturen zwischen 0 und 80°C, vorzugsweise jedoch bei Temperaturen zwischen 5 und 40°C, durchgeführt.

Bei Verwendung eines Äquivalents des verwendeten Oxidationsmittels erhält man bevorzugt die entsprechenden Sulfinylverbindungen und bei Verwendung von zwei Äquivalenten des verwendeten Oxidationsmittels die entsprechenden Sulfonylverbindungen.

Die gegebenenfalls anschließende Abspaltung eines verwendeten Schutzrestes erfolgt vorzugsweise hydrolytisch in einem wässrigen Lösungsmittel, z.B. in Wasser, Isopropanol/

Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches.

d) Zur Herstellung von Verbindungen der allgemeinen Formel I, in der X eine gegebenenfalls durch eine Alkylgruppe substituierte Iminogruppe, ein Sauerstoff- oder Schwefelatom darstellt:

Umsetzung einer Verbindung der allgemeinen Formel

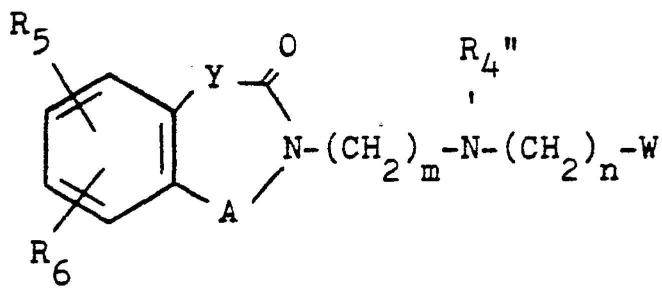
,(VII)

in der
A, $R_5$, $R_6$, Y, m und n wie eingangs definiert sind,

$R_4$" eine Schutzgruppe für eine Aminogruppe darstellt oder mit Ausnahme des Wasserstoffatoms die für $R_4$ eingangs erwähnten Bedeutungen besitzt und

W eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt, mit einer Verbindung der allgemeinen Formel

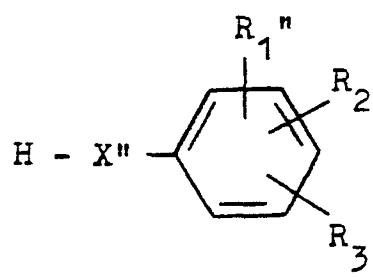
,(VIII)

in der
$R_2$ und $R_3$ wie eingangs definiert sind,
$R_1$" eine durch einen Schutzrest geschützte Amino-, Alkylamino- oder Hydroxygruppe, ein Wasserstoff-, Chlor- oder Bromatom, eine Dialkylamino- oder Alkoxygruppe darstellt, wobei jeder Alkylteil 1 bis 3 Kohlenstoffatome enthalten kann, und
X" eine gegebenenfalls durch eine Alkylgruppe mit 1 bis 3 Kohlenstoffatomen substituierte Iminogruppe, ein Sauerstoff- oder Schwefelatom bedeuten, und gegebenenfalls anschließende Abspaltung eines verwendeten Schutzrestes.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran oder Dioxan und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/

Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

e) Zur Herstellung von Verbindungen der allgemeinen Formel I, in der Y eine Carbonylgruppe darstellt:

Oxidation einer Verbindung der allgemeinen Formel

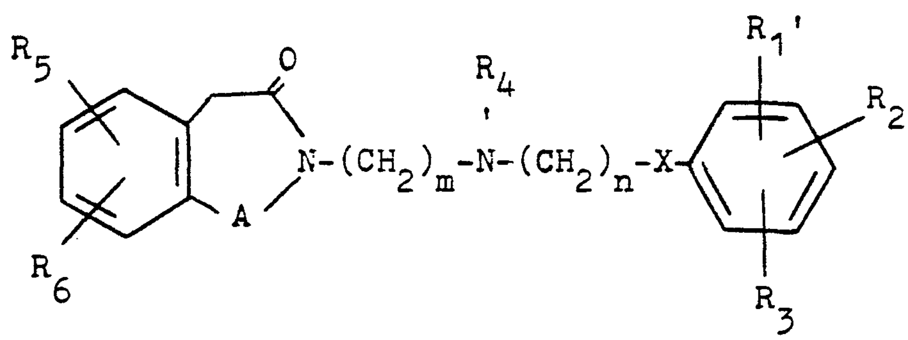

,(IX)

in der
A, $R_2$ bis $R_6$, X, m und n wie eingangs definiert sind und $R_1$' eine durch einen Schutzrest geschützte Hydroxy-, Amino- oder Alkylaminogruppe darstellt oder die für $R_1$ eingangs angegebenen Bedeutungen besitzt, und gegebenenfalls anschließende Abspaltung einer Schutzgruppe.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 10 und 100°C, vorzugsweise bei Temperaturen zwischen 20 und 80°C, durchgeführt. Falls X in der Verbindung der Formel I ein Schwefelatom oder eine Sulfinylgruppe darstellt, kann gleichzeitig Oxidation zu X = SO oder $SO_2$ erfolgen.

f) Zur Herstellung von Verbindungen der allgemeinen Formel I, in der Y eine gegebenenfalls durch eine Benzyl- oder Alkylgruppe substituierte Iminogruppe darstellt:

Umsetzung einer Verbindung der allgemeinen Formel

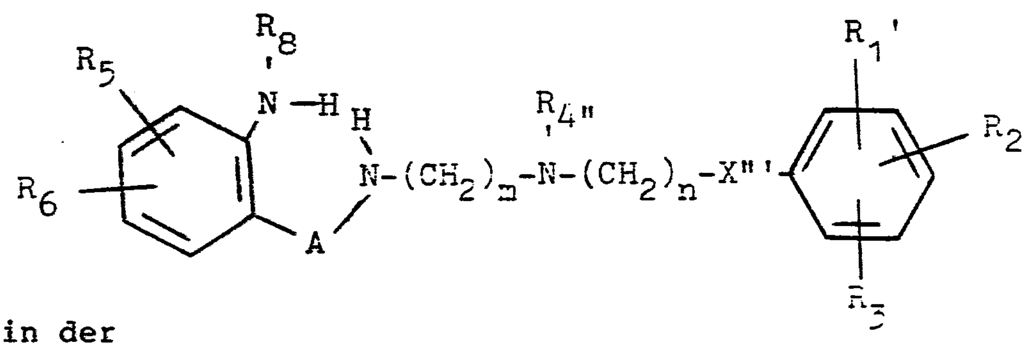

,(X)

in der

$R_2$, $R_3$, $R_5$, $R_6$, A, m und n wie eingangs definiert sind,

$R_1$' eine durch einen Schutzrest geschützte Hydroxy-, Amino- oder Alkylaminogruppe darstellt oder die für $R_1$ eingangs erwähnten Bedeutungen besitzt,

$R_4$" eine Schutzgruppe für eine Iminogruppe oder mit Ausnahme des Wasserstoffatoms die für $R_4$ eingangs erwähnten Bedeutungen besitzt,

X"' eine durch eine Schutzgruppe geschützte Iminogruppe oder mit Ausnahme der Iminogruppe die für X eingangs erwähnten Bedeutungen besitzt und

$R_8$ ein Wasserstoffatom, eine Benzylgruppe oder eine Alkylgruppe mit 1 bis 3 Kohlenstoffatomen bedeuten, mit einem Kohlensäurederivat der allgemeinen Formel

$$W - CO - W \qquad ,(XI)$$

in der
W, die gleich oder verschieden sein können, jeweils eine nukleophile Austrittsgruppe, z.B. ein Chlor- oder Bromatom, eine Alkoxygruppe mit 1 bis 3 Kohlenstoffatomen, eine Imidazolyl-(1)-gruppe oder auch eine Trichlormethoxygruppe, wenn der andere der Reste W ein Chlor- oder Bromatom darstellt, bedeuten, und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und
als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Essigester, Methylenchlorid, Tetrachlorkohlenstoff, Benzol, Tetrahydrofuran, Benzol/Tetrahydrofuran, Dioxan oder Acetonitril zweckmäßigerweise bei Temperaturen zwischen 0 und 150°C, vorzugsweise jedoch bei der Siedetemperatur des verwendeten Lösungsmittels, z.B. bei Temperaturen zwischen 40 und 100°C, und gegebenenfalls in Gegenwart eines säurebindenden Mittels wie Kaliumcarbonat, Natriumhydroxid, Kaliumhydroxid, Pyridin oder Triethylamin, wobei die letzteren gleichzeitig auch als Lösungsmittel dienen können, durchgeführt. Die Umsetzung kann jedoch auch ohne Lösungsmittel durchgeführt werden. Bedeutet in einer eingesetzten Verbindung der allgemeinen Formel XI mindestens einer der Reste W eine Alkoxygruppe mit 1 bis 3 Kohlenstoffatomen, so wird die Umsetzung vorzugsweise in einem Überschuß des eingesetzten Esters als Lösungsmittel durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/ Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes kann jedoch auch hydrogenolytisch erfolgen, z.B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

g) Zur Herstellung von Verbindungen der allgemeinen Formel I, in der $R_1$ eine Alkoxy-, Amino-, Alkylamino- oder Dialkylaminogruppe und mindestens einer der Reste $R_2$ oder $R_3$ ein Chlor- oder Bromatom darstellen:

Halogenierung einer Verbindung der allgemeinen Formel

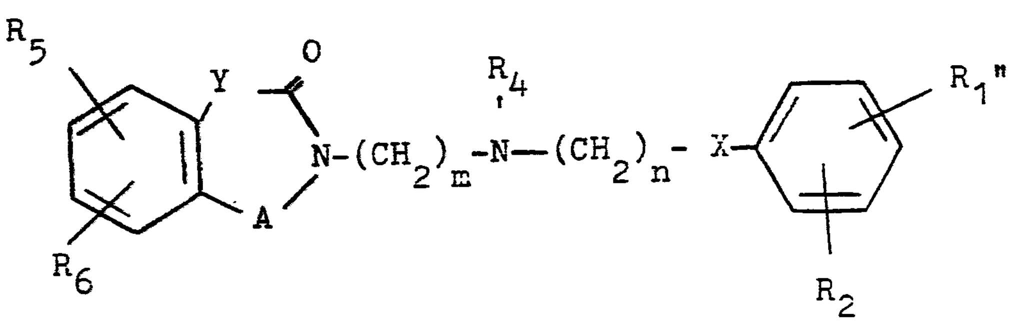

,(XII)

in der

A, $R_2$, $R_4$ bis $R_6$, X, Y, m und n wie eingangs definiert sind und

$R_1$" eine Alkoxy-, Amino-, Alkylamino- oder Dialkylaminogruppe darstellt.

Die Halogenierung wird vorzugsweise in einem geeigneten Lösungsmittel wie Eisessig, Methylenchlorid oder Dioxan in Gegenwart eines Halogenierungsmittels wie Chlor oder Brom bei Temperaturen zwischen 0 und 50°C, vorzugsweise jedoch bei Raumtemperatur, durchgeführt.

h) Zur Herstellung von Verbindungen der allgemeinen Formel I, in der $R_4$ ein Wasserstoffatom oder X eine Iminogruppe oder Y eine Iminogruppe oder $R_4$ ein Wasserstoffatom und X und/oder Y eine Iminogruppe darstellen:

Entbenzylierung einer Verbindung der allgemeinen Formel

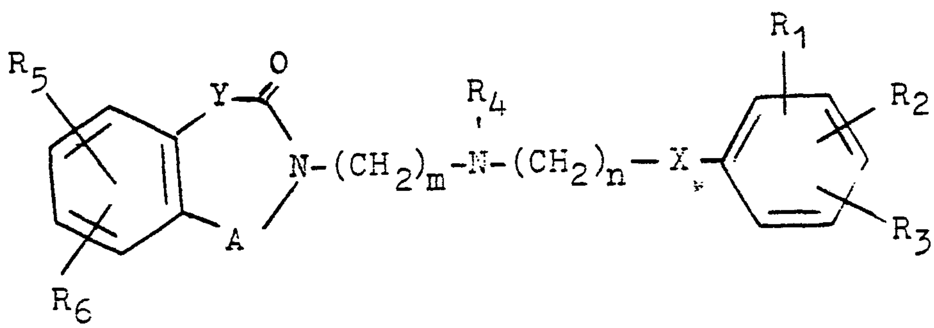

,(XIII)

in der
$R_1$ bis $R_6$, A, X, Y, m und n wie eingangs definiert sind, wobei jedoch $R_4$ eine Benzylgruppe oder X eine Benzyliminogruppe oder Y eine Benzyliminogruppe darstellen muß.

Die Entbenzylierung erfolgt vorzugsweise hydrogenolytisch, z.B. mit Wasserstoff in Gegenwart eines Katalysators wie Palladium/Kohle, in einem Lösungsmittel wie Methanol, Ethanol, Essisäureethylester oder Eisessig gegebenenfalls

unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Die erhaltenen Verbindungen der allgemeinen Formel I lassen sich in ihre Säureadditionssalze, insbesondere in ihre physiologisch verträgliche Säureadditionssalze mit anorganischen oder organischen Säuren überführen. Als Säuren kommen hierbei beispielsweise Salzäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XIII sind teilweise literaturbekannt bzw. man erhält sie nach an sich bekannten Verfahren.

So erhält man beispielsweise eine Ausgangsverbindung der allgemeinen Formel II durch Umsetzung eines entsprechenden Benzazepins mit einer entsprechenden Halogenverbindung und gegebenenfalls durch anschließende Umsetzung mit einem entsprechenden Amin. Das hierfür erforderliche in 3-Stellung unsubstituierte entsprechende Benzazepin erhält man durch Cyclisierung einer entsprechenden Verbindung, z.B. durch Cyclisierung einer Verbindung der allgemeinen Formel

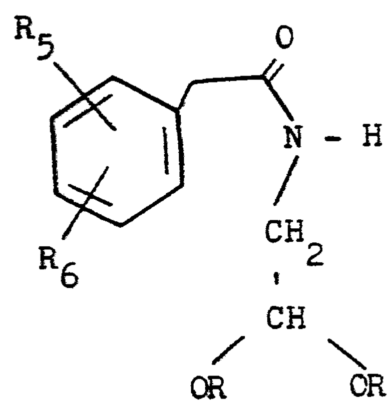

,(XIV)

und anschließender katalytischer Hydrierung und/oder Oxidation, z.B. mit Selendioxid.

Die als Ausgangsstoff verwendete Verbindung der allgemeinen Formel IX,X, XII oder XIII erhält man beispielsweise durch Umsetzung eines entsprechenden substituierten 1-Chlor-propans der allgemeinen Formel II mit einem entsprechenden Amin der allgemeinen Formel III.

Eine als Ausgangsstoff verwendete Verbindung der allgemeinen Formel IV erhält man vorzugsweise durch Umsetzung einer entsprechenden Halogenverbindung mit einem entsprechenden Amin und gegebenenfalls anschließende Abspaltung von Schutzresten, die zum Schutz von Hydroxy- und/oder Aminogruppen verwendet werden.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen Nebenwirkungen, z.B. einer geringen antimuskarinischen, eine lang anhaltende herzfrequenzsenkende Wirkung sowie eine Herabsetzung des $O_2$-Bedarfs des Herzens, außerdem weisen sie eine α-blockierende Wirkung auf.

Beispielsweise wurden die Verbindungen

A = N-[3-(7,8-Dimethoxy-1,3,4,5-tetrahydro-2H-3-benzazepin-2-on-3-yl)-propyl]-N-(2-phenylamino)-ethyl-methylamin,

B = N-[3-(7,8-Dimethoxy-1,3,4,5-tetrahydro-2H-3-benzazepin-2-on-3-yl)-propyl]-N-[2-(3,4-dimethoxyphenyloxy)-ethyl]-methylamin
und

C = N-[3-(7,8-Dimethoxy-1,3,4,5-tetrahydro-2H-3-benzazepin-2-on-3-yl)-propyl]-N-(3-phenylthio-propyl)-methylamin

auf ihre biologischen Eigenschaften hin untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/100 g).

Der Blutdruck wurde über eine in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung, gemessen 20 Minuten nach Substanzapplikation [S/min] |
|---|---|---|
| A | 5,0 | - 160 |
| B | 5,0 | - 164 |
| C | 5,0 | - 107 |

Die erfindunsgemäß hergestellten Verbindungen weisen eine gute Vertäglichkeit auf, da bei den obigen Untersuchungen keine toxischen Nebenwirkungen beobachtet werden konnten.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/ Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Herstellung der Ausgangsverbindungen:

Beispiel A

7,8-Dimethoxy-1,3-dihydro-2H-3-benzazepin-2-on

a) 3,4-Dimethoxy-phenylessigsäurechlorid

Zu einer Suspension von 549,4g 3,4-Dimethoxy-phenylessigsäure in 600 ml Methylenchlorid werden während 2 Stunden 600 ml Thionylchlorid unter Rühren zugetropft. Nach beendeter Gasentwicklung (16 Stunden) wird noch eine Stunde am Rückfluß gekocht. Nach dem Entfernen der leicht flüchtigen Komponenten wird der Rückstand im Vakuum destilliert.
Ausbeute: 486 g (80,8 % der Theorie),
Kp: 134-136°C/1,95 mbar

b) N-(2,2-Dimethoxyethyl)-3,4-dimethoxy-phenylacetamid

Unter Eiskühlung wird eine Lösung von 485,2 g 3,4-Dimethoxy-phenylessigsäurechlorid in 1,1 l Methylenchlorid bei 15-20°C zu einer Lösung von 246,2 ml Aminoacetaldehyddimethylacetal und 315 ml Triethylamin in 2,2 l Methylenchlorid zugetropft und eine Stunde bei 16-18°C nachgerührt. Anschließend wird mehrfach mit Wasser extrahiert, über Magnesiumsulfat getrocknet und eingedampft. Das erhaltende Öl kristallisiert langsam durch.
Ausbeute: 608 g (95 % der Theorie),
Schmelzpunkt: 66-69°C.

c) 7,8-Dimethoxy-1,3-dihydro-2H-3-benzazepin-2-on

Eine Lösung von 600,6 g N-(2,2-Dimethoxyethyl)-3,4-dimethoxy-phenylacetamid in 3 l konzentrierter Salzsäure wird mit 3 l Eisessig versetzt. Nach 17-stündigem Stehenlassen bei

Raumtemperatur wird der Ansatz auf Eis gegossen. Die ausgefallenen Kristalle werden abgesaugt, mit Wasser neutral gewaschen und getrocknet.
Ausbeute: 350 g (75,4 % der Theorie),
Schmelzpunkt: 234-237°C.

Beispiel B

1-(7,8-Dimethoxy-1,3,4,5-tetrahydro-2H-3-benzazepin-2-on-3-yl)-3-chlor-propan

a) 1-(7,8-Dimethoxy-1,3-dihydro-2H-3-benzazepin-2-on-3-yl)-3-chlor-propan

131,5 g (0,6 Mol) 7,8-Dimethoxy-1,3-dihydro-2H-3-benzazepin-2-on werden in 900 ml Dimethylsulfoxid suspendiert und unter Rühren mit 80,8 g (0,72 Mol) Kalium-tert.butylat versetzt. Nach 10 Minuten wird die erhaltene Lösung unter Kühlung mit Eiswasser zu 77 ml (0,72 Mol) 1-Brom-3-chlorpropan in 300 ml Dimethylsulfoxid getropft. Nach einer Stunde gießt man auf Eiswasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.

Ausbeute: 155,5 g (87,3 % der Theorie),
Schmelzpunkt: 101-103°C

b) 1-(7,8-Dimethoxy-1,3,4,5-tetrahydro-2H-3-benzazepin-2-on-3-yl)-3-chlor-propan

59,2 g (0,2 Mol) 1-(7,8-Dimethoxy-1,3-dihydro-2H-3-benzazepin-2-on-3-yl)-3-chlor-propan werden in 500 ml Eisessig in Anwesenheit von 5 g 10%iger Palladium-Kohle 6 Stunden bei 50°C und 5 bar hydriert. Der Katalysator wird abgesaugt, der

Eisessig im Vakuum abdestilliert und der Rückstand nach Zugabe von Wasser mit Kaliumcarbonat neutralisiert. Der Niederschlag wird abgesaugt, mit Wasser salzfrei gewaschen und getrocknet.
Ausbeute: 53 g (89 % der Theorie),
Schmelzpunkt: 85-86°C

Beispiel C

N-[3-(7,8-Dimethoxy-1,3,4,5-tetrahydro-2H-3-benzazepin-2-on-3-yl)-propyl]-methylamin

a) N-[3-(7,8-Dimethoxy-1,3,4,5-tetrahydro-2H-3-benzazepin-2-on-3-yl)-propyl]-N-methyl-benzylamin

60 g (0,2 Mol) 3-(7,8-Dimethoxy-1,3,4,5-tetrahydro-2H-3-benzazepin-2-on-3-yl)-1-chlorpropan werden mit 65 g (0,54 Mol) N-Methyl-benzylamin 1,5 Stunden lang auf 130°C erhitzt. Nach Abkühlen wird zwischen Wasser und Methylenchlorid verteilt; die organische Phase wird abgetrennt, getrocknet und eingeengt. Der Rückstand wird durch Chromatographie an Kieselgel gereinigt (Elutionsmittel: Methylenchlorid/Methanol = 10:1).
Ausbeute: 97,4 % der Theorie,
Schmelzpunkt des Hydrochlorids: 205-208°C

b) N-[3-(7,8-Dimethoxy-1,3,4,5-tetrahydro-2H-3-benzazepin-2-on-3-yl)-propyl]-methylamin

72,5 g (0,19 Mol) N-[3-(7,8-Dimethoxy-1,3,4,5-tetrahydro-2H-3-benzazepin-2-on-3-yl)-propyl]-N-methyl-benzylamin werden in Eisessiglösung bei Raumtemperatur in Gegenwart von Palladium/Kohle und 5 bar Wasserstoff entbenzyliert.
Ausbeute: 48,7 g (87,7 % der Theorie),
Schmelzpunkt des Hydrochlorids: 132-138°C.

Beispiel D

N-[3-(7,8-Dimethoxy-1,3,4,5-tetrahydro-2H-3-benzazepin-2-on-3-yl)-propyl]-N-(3-chlorpropyl)-methylamin

5 g (0,017 Mol) N-[3-(7,8-Dimethoxy-1,3,4,5-tetrahydro-2H-3-benzazepin-2-on-3-yl)-propyl]-methylamin werden mit 40 ml 1-Brom-3-chlorpropan 30 Minuten lang auf 120°C erwärmt. Man engt i.V. zur Trockne ein, verteilt den Rückstand zwischen Natronlauge und Methylenchlorid, trennt die organische Phase ab, engt sie ein und reinigt den Rückstand durch Chromatographie an Kieselgel (Elutionsmittel: Methylenchlorid/Methanol = 10/1).

Ausbeute: 2 g (31,9 %, Öl).

Spektren: IR (Methylenchlorid): 1650 $cm^{-1}$ (Lactam-CO)

MS: $M^+$ 368/370 m/e (Mono-Chlor)

Herstellung der Endprodukte:

Beispiel 1

N-[3-(7,8-Dimethoxy-1,3,4,5-tetrahydro-2H-3-benzazepin-2-on-3-yl)-propyl]-2-(3,4-dimethoxyphenylamino)-ethylamin-dihydrochlorid

10 g (0,0336 Mol) 3-(7,8-Dimethoxy-1,3,4,5-tetrahydro-2H-3-benzazepin-2-on-3-yl)-1-chlorpropan werden mit 13 g (0,066 Mol) 2-(3,4-Dimethoxyphenylamino)-ethylamin 3 Stunden lang auf 130°C erhitzt. Nach Abkühlen wird das Reaktionsgemisch zwischen Natronlauge und Methylenchlorid verteilt, die organische Phase abgetrennt, über Natriumsulfat getrocknet und eingeengt; der Rückstand wird an Kieselgel chromatographiert (Elutionsmittel: Methylenchlorid/Methanol/konz. Ammoniak: 90/10/0,25). Das so erhaltene Produkt wird auf üblichem Wege in das Dihydrochlorid überführt.
Ausbeute: 8,12 g (45,5 % der Theorie),
Schmelzpunkt: 205-209°C (Zers.)

Beispiel 2

N-[3-(7,8-Dimethoxy-1,3,4,5-tetrahydro-2H-3-benzazepin-2-on-3-yl)-propyl]-N-(2-phenylaminoethyl)-methylamin

9 g (0,0336 Mol) 3-(7,8-Dimethoxy-1,3,4,5-tetrahydro-2H-3-benzazepin-2-on-3-yl)-1-chlorpropan werden mit 11 g (0,073 Mol) N-(2-Phenylaminoethyl)-methylamin 30 Minuten lang auf 130°C erhitzt. Nach dem Abkühlen wird das Reaktionsgemisch zwischen Wasser und Diethylether verteilt,die

organische Phase wird abgetrennt, über Natriumsulfat getrocknet und eingeengt. Der Rückstand wird aus Toluol/Petroläther umkristallisiert.
Ausbeute: 8,7 g (71 % der Theorie),
Schmelzpunkt: 75-78°C

Beispiel 3

N-[3-(7,8-Dimethoxy-1,3,4,5-tetrahydro-2H-3-benzazepin-2-on-3-yl)-propyl]-N-[2-(3,4-dimethoxyphenylthio)-ethyl]-methylamin

---

0,3 g (0,00102 Mol) N-[3-(7,8-Dimethoxy-1,3,4,5-tetrahydro-2H-3-benzazepin-2-on-3-yl)-propyl]-methylamin werden mit 0,24 g (0,00103 Mol) 2-(3,4-Dimethoxy-phenylthio)-ethylchlorid 5 Stunden lang auf 100-120°C erhitzt. Das Reaktionsgemisch wird zwischen Wasser und Methylenchlorid verteilt, die organische Phase wird abgetrennt, über Natriumsulfat getrocknet und eingeengt. Der Rückstand wird durch Chromatographie an Kieselgel (Elutionsmittel: Methylenchlorid/Methanol = 9/1) gereinigt.
Ausbeute: 160 mg Öl (32,6 % der Theorie),
Spektren: IR (Methylenchlorid): 1650 $cm^{-1}$ (Lactam-CO)
MS: $M^+$ 488 m/e

Beispiel 4

N-[3-(7,8-Dimethoxy-1,3,4,5-tetrahydro-2H-3-benzazepin-2-on-3-yl)-propyl]-N-(3-phenylsulfinyl-propyl)-methylamin

---

440 mg (0,001 Mol) N-[3-(7,8-Dimethoxy-1,3,4,5-tetrahydro-2H-3-benzazepin-2-on-3-yl)-propyl]-N-(3-phenylthio-propyl)-methylamin werden in 2,5 ml Eisessig und 1,1 ml 30%igem Wasserstoffperoxid 2 Stunden lang bei Raumtemperatur gerührt. Das Reaktionsgemisch wird auf Eis gegossen, mit Ammoniak alkalisch gestellt und mit Methylenchlorid extrahiert. die organische Phase wird über Natriumsulfat getrocknet und eingeengt.
Ausbeute: 0,45 g (Öl, 98 % der Theorie),
Spektren: IR (Methylenchlorid): 1650 $cm^{-1}$ (Lactam-CO)
MS: $M^+$ 458 m/e

Beispiel 5

N-[3-(7,8-Dimethoxy-1,3,4,5-tetrahydro-2H-3-benzazepin-2-on-3-yl)-propyl]-N-[2-(N-phenyl-methylamino)-ethyl]-methylamin

1 g (0,0024 Mol) N-[3-(7,8-Dimethoxy-1,3,4,5-tetrahydro-2H-3-benzazepin-2-on-3-yl)-propyl]-N-(2-phenylamino-ethyl)-methylamin werden in 20 ml Äthanol mit 0,3 g Paraformaldehyd und 0,62 g (0,01 Mol) Natriumcyanborhydrid gerührt, wobei der pH-Wert des Gemisches durch Zugabe von Salzsäure zwischen 6,5 und 7 gehalten wird. Nach Beendigung der Reaktion wird überschüssiges Natriumcyanborhydrid durch Zugabe von Salzsäure bei pH 1 zerstört; das Gemisch wird alkalkisch gestellt und mit Methylenchlorid extrahiert; die organische Phase wird abgetrennt, mit Wasser gewaschen, über Natriumsulfat getrocknet und eingeengt. Der Rückstand wird durch Chromatographie an Kieselgel (Elutionsmittel: Methylenchlorid/Methanol/konz. Ammoniak = 90/10/0,85) gereinigt.
Ausbeute: 0,35 g feste Substanz (34 % der Theorie),
Spektren: IR (Methylenchlorid): 1650 $cm^{-1}$ (Lactam-CO)
MS: $M^+$ 425 m/e

Beispiel 6

N-[3-(7,8-Dimethoxy-1,3,4,5-tetrahydro-2H-3-benzazepin-2-on-3-yl)-propyl]-N-(2-phenyloxyethyl)-methylamin-hydrochlorid

18,5 g (0,0623 Mol) 3-(7,8-Dimethoxy-1,3,4,5-tetrahydro-2H-3-benzazepin-2-on-3-yl)-1-chlorpropan werden in einem Autoklaven mit 10,3 g (0,0623 Mol) N-(2-Phenyloxyethyl)-methylamin und 8,7 ml (0,062 Mol) Triethylamin 2 Stunden lang auf 110°C erhitzt. Nach Abkühlen wird das Reaktionsgemisch zwischen Methylenchlorid und Wasser verteilt, die organische Phase wird abgetrennt, über Natriumsulfat getrocknet und eingeengt. Der Rückstand wird durch Chromatographie an Kieselgel (Elutionsmittel: Methylenchlorid/Methanol = 9/1) gereinigt. Die so erhaltene Base wird auf üblichem Wege in das Hydrochlorid überführt.
Ausbeute: 12,7 g (44 % der Theorie)
Schmelzpunkt: 177-179°C

Beispiel 7

N-[3-(7,8-Dimethoxy-1,3,4,5-tetrahydro-2H-3-benzazepin-2-on-3-yl)-propyl]-N-[3-(4-amino-3,5-dibromphenylsulfonyl)-propyl]-methylamin

0,8 g (0,0013 Mol) N-[3-(7,8-Dimethoxy-1,3,4,5-tetrahydro-2H-3-benzazepin-2-on-3-yl)-propyl]-N-[3-(4-amino-3,5-dibromphenylthio)-propyl]-methylamin werden in 10 ml Eisessig gelöst und mit 2 ml 30%igem Wasserstoffperoxid vermischt. Nach 2 Stunden langem Rühren bei Raumtemperatur werden weitere 5 ml Wasserstoffperoxid zugesetzt und nach insgesamt 3 Stunden nochmals 5 ml. Das Reaktionsgemisch wird nach weiteren 15 Stunden auf Eis gegossen, mit Natronlauge alkalisch gestellt und mit Methylenchlorid extrahiert. Die organische Phase wird über Natriumsulfat getrocknet und eingeengt. Der Rückstand wird durch Chromatographie an Kieselgel (Elutionsmittel: Methylenchlorid/Methanol = 10/1) gereinigt.
Ausbeute: 0,65 g (77,4 % der Theorie),
Spektren: IR (Methylenchlorid): 1650 $cm^{-1}$ (Lactam-CO)
MS: $M^+$ 645/647/649 m/e ($Br_2$)

Beispiel 8

N-[3-(7,8-Dimethoxy-1,3,4,5-tetrahydro-2H-3-benzazepin-2-on-3-yl)-propyl]-N-[3-(4-acetaminophenylthio)-propyl]-methylamin

0,72 g (0,002 Mol) N-[3-(7,8-Dimethoxy-1,3,4,5-tetrahydro-2H-3-benzazepin-2-on-3-yl)-propyl]-N-(3-chlorpropyl)-methylamin werden mit einer Lösung von 0,42 g (0,0025 Mol) 4-Acetaminothiophenol und 0,175 g (0,0025 Mol) Kaliummethylat in 20 ml Methanol 2 Stunden lang gekocht. Dann wird das Reaktionsgemisch zur Trockne eingeengt, zwischen Natronlauge und Methylenchlorid verteilt; die organische Phase wird abgetrennt, getrocknet und eingeengt. Der Rückstand wird über Kieselgel chromatographiert (Elutionsmittel: Methylenchlorid/Methanol = 10/1). Man erhält 600 mg = 60 % eines harzartigen Produktes.
Spektren: IR (Methylenchlorid): 1650 $cm^{-1}$ (Lactam-CO)
MS: $M^+$ 499 m/e

Beispiel 9

N-[3-(7,8-Dimethoxy-1,3,4,5-tetrahydro-2H-3-benzazepin-2-on-3-yl)-propyl]-N-[3-(3,4-dimethoxyphenylthio)-propyl]-methylamin

Hergestellt aus N-[3-(7,8-Dimethoxy-1,3,4,5-tetrahydro-2H-3-benzazepin-2-on-3-yl)-propyl]-methylamin und 3-(3,4-Dimethoxyphenylthio)-propylchlorid unter Zusatz von Pyridin analog Beispiel 3.
Ausbeute: 34,4 % der Theorie,
Spektren: IR (Methylenchlorid): 1650 $cm^{-1}$ (Lactam-CO)
MS: $M^+$ 502/m/e
Das Hydrochlorid hat einen Schmelzpunkt von 150-154°C.

Beispiel 10

N-[3-(7,8-Dimethoxy-1,3,4,5-tetrahydro-2H-3-benzazepin-2-on-3-yl)-propyl]-N-(3-phenylthiopropyl)-methylamin

Hergestellt aus N-[3-(7,8-Dimethoxy-1,3,4,5-tetrahydro-2H-3-benzazepin-2-on-3-yl)-propyl]-methylamin und 3-Phenylthiopropylchlorid analog Beispiel 3.
Ausbeute: 30,5 % der Theorie,
Schmelzpunkt des Hydrochlorids: 130-140°C

Beispiel 11

N-[3-(7,8-Dimethoxy-1,3,4,5-tetrahydro-2H-3-benzazepin-2-on-3-yl)-propyl]-N-(2-phenylthioethyl)-methylamin

Hergestellt aus N-[3-(7,8-Dimethoxy-1,3,4,5-tetrahydro-2H-3-benzazepin-2-on-3-yl)-propyl]-methylamin und 2-Phenylthioethylchlorid analog Beispiel 3.

Ausbeute: 23,3 % der Theorie,

Spektren: IR (Methylenchlorid): 1650 $cm^{-1}$ (Lactam-CO)

MS: $M^+$ 428 m/e

Das Hydrochlorid hat einen Schmelzpunkt von 110-120°C

Beispiel 12

N-[3-(7,8-Dimethoxy-1,3,4,5-tetrahydro-2H-3-benzazepin-2-on-3-yl)-propyl]-N-(2-phenylsulfinylethyl)-methylamin

Hergestellt aus N-[3-(7,8-Dimethoxy-1,3,4,5-tetrahydro-2H-3-benzazepin-2-on-3-yl)-propyl]-N-(2-phenylthioethyl)-methylamin und Wasserstoffperoxid analog Beispiel 4.

Ausbeute: 89,9 % der Theorie,

Spektren: IR (Methylenchlorid): 1650 $cm^{-1}$ (Lactam-CO)

MS: $M^+$ 444 m/e

Beispiel 13

N-[3-(7,8-Dimethoxy-1,3,4,5-tetrahydro-2H-3-benzazepin-2-on-3-yl)-propyl]-N-[3-(3,4-dimethoxyphenylsulfinyl)-propyl]-methylamin

Hergestellt aus N-[3-(7,8-Dimethoxy-1,3,4,5-tetrahydro-2H-3-benzazepin-2-on-3-yl)-propyl]-N-[3-(3,4-dimethoxyphenylthio)-propyl]-methylamin und Wasserstoffperoxid analog Beispiel 4.

Ausbeute: 66,6 % der Theorie,

Spektren: IR (Methylenchlorid): 1650 $cm^{-1}$ (Lactam-CO)

MS: $M^+$ 518 m/e

0161599

Beispiel 14

N-[3-(7,8-Dimethoxy-1,3,4,5-tetrahydro-2H-3-benzazepin-2-on-3-yl)-propyl]-N-[2-(3,4-dimethoxyphenylsulfinyl)-ethyl]-methylamin

Hergestellt aus N-[3-(7,8-Dimethoxy-1,3,4,5-tetrahydro-2H-3-benzazepin-2-on-3-yl)-propyl]-N-[2-(3,4-dimethoxyphenylthio)-ethyl]-methylamin und Wasserstoffperoxid analog Beispiel 4.

Ausbeute: 90 % der Theorie,

Spektren: IR (Methylenchlorid): 1650 $cm^{-1}$ (Lactam-CO)

MS: $M^+$ 504 m/e

Beispiel 15

N-[3-(7,8-Dimethoxy-1,3,4,5-tetrahydro-2H-3-benzazepin-2-on-3-yl)-propyl]-N-[2-(N-3,4-dimethoxyphenyl-N-methyl-amino)-ethyl]-methylamin

Hergestellt aus N-[3-(7,8-Dimethoxy-1,3,4,5-tetrahydro-2H-3-benzazepin-2-on-3-yl)-propyl]-2-(3,4-dimethoxyphenylamino)-ethylamin, Paraformaldehyd und Natriumcyanborhydrid analog Beispiel 5.

Ausbeute: 79,5 % der Theorie,

Spektren: IR (Methylenchlorid): 1650 $cm^{-1}$ (Lactam-CO)

MS: $M^+$ 485 m/e

Beispiel 16

N-[3-(7,8-Dimethoxy-1,3,4,5-tetrahydro-2H-3-benzazepin-2-on-3-yl)-propyl]-3-(phenylamino)-propylamin-dihydrochlorid

Hergestellt aus 3-(7,8-Dimethoxy-1,3,4,5-tetrahydro-2H-3-benzazepin-2-on-3-yl)-1-chlorpropan und 3-Phenylamino-propylamin in Gegenwart von Triethylamin analog Beispiel 1.
Ausbeute: 6,8 % der Theorie,
Schmelzpunkt: 224-226°C (Zers.)

Beispiel 17

N-[3-(7,8-Dimethoxy-1,3,4,5-tetrahydro-2H-3-benzazepin-2-on-3-yl)-propyl]-N-(3-phenylaminopropyl)-methylamin

Hergestellt aus N-[3-(7,8-Dimethoxy-1,3,4,5-tetrahydro-2H-3-benzazepin-2-on-3-yl)-propyl]-3-phenylamino-propylamin, Paraformaldehyd und Natriumcyanborhydrid analog Beispiel 5.
Ausbeute: 52,6 % der Theorie,
Spektren: IR (Methylenchlorid): 1650 $cm^{-1}$ (Lactam-CO)
MS: $M^+$ 425 m/e

Beispiel 18

N-[3-(7,8-Dimethoxy-1,3,4,5-tetrahydro-2H-3-benzazepin-2-on-3-yl)-propyl]-3-(3,4-dimethoxyphenylamino)-propylamin-hydrochlorid

Hergestellt aus 3-(7,8-Dimethoxy-1,3,4,5-tetrahydro-2H-3-

benzazepin-2-on-3-yl)-1-chlorpropan und 3-(3,4-Dimethoxy-phenylamino)-propylamin analog Beispiel 1.
Ausbeute: 34,6 % der Theorie,
Schmelzpunkt: 206-212°C (Zers.)

## Beispiel 19

N-[3-(7,8-Dimethoxy-1,3,4,5-tetrahydro-2H-3-benzazepin-2-on-3-yl)-propyl]-N-[3-(4-amino-3,5-dibromphenylthio)-propyl]-methylamin

Hergestellt aus N-[3-(7,8-Dimethoxy-1,3,4,5-tetrahydro-2H-3-benzazepin-2-on-3-yl)-propyl]-methylamin und 3-(4-Amino-3,5-dibromphenylthio)-propylchlorid analog Beispiel 3.
Ausbeute: 16,2 % der Theorie,
Spektren: IR (Methylenchlorid): 1650 $cm^{-1}$ (Lactam-CO)
MS: $M^+$ 613/615/617 ($Br_2$)

## Beispiel 20

N-[3-(7,8-Dimethoxy-1,3,4,5-tetrahydro-2H-3-benzazepin-2-on-3-yl)-propyl]-N-[3-(4-amino-3,5-dibromphenylsulfinyl)-propyl]-methylamin

Hergestellt aus N-[3-(7,8-Dimethoxy-1,3,4,5-tetrahydro-2H-3-benzazepin-2-on-3-yl)-propyl]-N-[3-(4-amino-3,5-dibromphenylthio)-propyl]-methylamin und Wasserstoffperoxid in Eisessig analog Beispiel 4.
Ausbeute: 60 % der Theorie,
Spektren: IR (Methylenchlorid): 1650 $cm^{-1}$ (Lactam-CO)
MS: $M^+$ 629/631/633 ($Br_2$)

Beispiel 21

N-[3-(7,8-Dimethoxy-1,3,4,5-tetrahydro-2H-3-benzazepin-2-on-3-yl)-propyl]-2-(4-amino-3,5-dichlorphenylamino)-ethylamin-dihydrochlorid

---

Hergestellt aus 3-(7,8-Dimethoxy-1,3,4,5-tetrahydro-2H-3-benzazepin-2-on-3-yl)-1-chlorpropan und 2-(4-Amino-3,5-dichlorphenylamino)-ethylamin analog Beispiel 1.
Ausbeute: 46 % der Theorie,
Schmelzpunkt: 226-232°C (Zers.)

Beispiel 22

N-[3-(7,8-Dimethoxy-1,3,4,5-tetrahydro-2H-3-benzazepin-2-on-3-yl)-propyl]-3-(4-amino-3,5-dichlorphenylamino)-propylamin-dihydrochlorid

---

Hergestellt aus 3-(7,8-Dimethoxy-1,3,4,5-tetrahydro-2H-3-benzazepin-2-on-3-yl)-1-chlorpropan und 3-(4-Amino-3,5-dichlorphenylamino)-propylamin analog Beispiel 1.
Ausbeute: 45 % der Theorie,
Schmelzpunkt: 218-220°C (Zers.)

Beispiel 23

N-[3-(7,8-Dimethoxy-1,3,4,5-tetrahydro-2H-3-benzazepin-2-on-3-yl)-propyl]-2-(4-dimethylaminophenylamino)-ethylamin-dihydrochlorid

---

Hergestellt aus 3-(7,8-Dimethoxy-1,3,4,5-tetrahydro-2H-3-

benzazepin-2-on-3-yl)-1-chlorpropan und 2-(4-Dimethylamino-phenylamino)-ethylamin analog Beispiel 1.
Ausbeute: 16 % der Theorie,
Schmelzpunkt: 214-218°C (Zers.)

Beispiel 24

N-[3-(7,8-Dimethoxy-1,3,4,5-tetrahydro-2H-3-benzazepin-2-on-3-yl)-propyl]-3-(4-dimethylaminophenylamino)-propylamin-hydrochlorid

Hergestellt aus 3-(7,8-Dimethoxy-1,3,4,5-tetrahydro-2H-3-benzazepin-2-on-3-yl)-1-chlorpropan und 3-(4-Dimethylamino-phenylamino)-propylamin analog Beispiel 1.
Ausbeute: 18,5 % der Theorie,
Schmelzpunkt: 205-208°C (Zers.)

Beispiel 25

N-[3-(7,8-Dimethoxy-1,3,4,5-tetrahydro-2H-3-benzazepin-2-on-3-yl)-propyl]-2-(4-amino-3,5-dibromphenylamino)-ethylamin-hydrochlorid

Hergestellt aus 3-(7,8-Dimethoxy-1,3,4,5-tetrahydro-2H-3-benzazepin-2-on-3-yl)-1-chlorpropan und 2-(4-Amino-3,5-dibromphenylamino)-ethylamin analog Beispiel 1.
Ausbeute: 39 % der Theorie,
Schmelzpunkt: 225-230°C (Zers.)

Beispiel 26

N-[3-(7,8-Dimethoxy-1,3,4,5-tetrahydro-2H-3-benzazepin-2-on-3-yl)-propyl]-3-(4-amino-3,5-dibromphenylamino)-propylamin-hydrochlorid

---

Hergestellt aus 3-(7,8-Dimethoxy-1,3,4,5-tetrahydro-2H-3-benzazepin-2-on-3-yl)-1-chlorpropan und 3-(4-Amino-3,5-dibromphenylamino)-propylamin analog Beispiel 1.
Ausbeute: 22 % der Theorie,
Schmelzpunkt: 208-212°C (Zers.)

Beispiel 27

N-[3-(7,8-Dimethoxy-1,3,4,5-tetrahydro-2H-3-benzazepin-2-on-3-yl)-propyl]-N-[3-(3,4-dimethoxyphenyloxy)-propyl]-methylamin-hydrochlorid

---

Hergestellt aus 3-(7,8-Dimethoxy-1,3,4,5-tetrahydro-2H-3-benzazepin-2-on-3-yl)-1-chlorpropan und N-Methyl-3-(3,4-dimethoxyphenyloxy)-propylamin analog Beispiel 6.
Ausbeute: 6,5 % der Theorie,
Schmelzpunkt: 179-183°C

Beispiel 28

N-[3-(7,8-Dimethoxy-1,3,4,5-tetrahydro-2H-3-benzazepin-2-on-3-yl)-propyl]-N-[4-(3,4-dimethoxyphenyloxy)-butyl]-methylamin-hydrochlorid

---

Hergestellt aus 3-(7,8-Dimethoxy-1,3,4,5-tetrahydro-2H-3-

benzazepin-2-on-3-yl)-1-chlorpropan und N-Methyl-4-(3,4-dimethoxyphenyloxy)-butylamin analog Beispiel 6.
Ausbeute: 13,7 % der Theorie,
Schmelzpunkt: 162-166°C (Zers.)

Beispiel 29

N-[3-(7,8-Dimethoxy-1,3,4,5-tetrahydro-2H-3-benzazepin-2-on-3-yl)-propyl]-N-[2-(3,4-methylendioxyphenyloxy)-ethyl]-methylamin-hydrochlorid

Hergestellt aus 3-(7,8-Dimethoxy-1,3,4,5-tetrahydro-2H-3-benzazepin-2-on-3-yl)-1-chlorpropan und N-Methyl-2-(3,4-methylendioxyphenyloxy)-ethylamin analog Beispiel 6.
Ausbeute: 17,4 % der Theorie,
Schmelzpunkt: 199-202°C

Beispiel 30

N-[3-(7,8-Dimethoxy-1,3,4,5-tetrahydro-2H-3-benzazepin-2-on-3-yl)-propyl]-N-[2-(4-methoxyphenyloxy)-ethyl]-methylamin-hydrochlorid

Hergestellt aus 3-(7,8-Dimethoxy-1,3,4,5-tetrahydro-2H-3-benzazepin-2-on-3-yl)-1-chlorpropan und N-Methyl-2-(4-methoxyphenyloxy)-ethylamin analog Beispiel 6.
Ausbeute: 39,1 % der Theorie,
Schmelzpunkt: 194-196°C

Beispiel 31

N-[3-(7,8-Dimethoxy-1,3,4,5-tetrahydro-2H-3-benzazepin-2-on-3-yl)-propyl]-N-[2-(3,4-dichlorphenyloxy)-ethyl]-methylamin-hydrochlorid

Hergestellt aus 3-(7,8-Dimethoxy-1,3,4,5-tetrahydro-2H-3-benzazepin-2-on-3-yl)-1-chlorpropan und N-Methyl-2-(3,4-dichlorphenyloxy)-ethylamin analog Beispiel 6.
Ausbeute: 36,6 % der Theorie,
Schmelzpunkt: 185-187°C

Beispiel 32

N-[3-(7,8-Dimethoxy-1,3,4,5-tetrahydro-2H-3-benzazepin-2-on-3-yl)-propyl]-N-[3-(3,4-dimethoxyphenylsulfonyl)-propyl]-methylamin

Hergestellt aus N-[3-(7,8-Dimethoxy-1,3,4,5-tetrahydro-2H-3-benzazepin-2-on-3-yl)-propyl]-N-[3-(3,4-dimethoxyphenylthio)-propyl]-methylamin und Wasserstoffperoxid analog Beispiel 7.
Ausbeute: 73,3 % der Theorie,
Spektren: IR (Methylenchlorid): 1650 $cm^{-1}$ (Lactam-CO)
MS: $M^+$ 534 m/e

Beispiel 33

N-[3-(7,8-Dimethoxy-1,3,4,5-tetrahydro-2H-3-benzazepin-2-on-3-yl)-propyl]-N-(3-phenylsulfonylpropyl)-methylamin

Hergestellt aus N-[3-(7,8-Dimethoxy-1,3,4,5-tetrahydro-2H-3-benzazepin-2-on-3-yl)-propyl]-N-(3-phenylthiopropyl)-methylamin und Wasserstoffperoxid analog Beispiel 7.
Ausbeute: 94,7 % der Theorie,
Spektren: IR (Methylenchlorid): 1650 $cm^{-1}$ (Lactam-CO)
MS: $M^+$ 474/me

Beispiel 34

N-[3-(7,8-Dimethoxy-1,3,4,5-tetrahydro-2H-3-benzazepin-2-on-3-yl)-propyl]-N-(2-phenylsulfonylethyl)-methylamin

Hergestellt aus N-[3-(7,8-Dimethoxy-1,3,4,5-tetrahydro-2H-3-benzazepin-2-on-3-yl)-propyl]-N-(2-phenylthioethyl)-methylamin und Wasserstoffperoxid analog Beispiel 7.
Ausbeute: 47,8 % der Theorie,
Spektren: IR (Methylenchlorid): 1650 $cm^{-1}$ (Lactam-CO)
MS: $M^+$ 460 m/e

Beispiel 35

N-[3-(7,8-Dimethoxy-1,3,4,5-tetrahydro-2H-3-benzazepin-2-on-3-yl)-propyl]-N-[2-(3,4-dimethoxyphenylsulfonyl)-ethyl]-methylamin

Hergestellt aus N-[3-(7,8-Dimethoxy-1,3,4,5-tetrahydro-2H-3-benzazepin-2-on-3-yl)-propyl]-N-[2-(3,4-dimethoxyphenylthio)-ethyl]-methylamin und Wasserstoffperoxid analog Beispiel 7.
Ausbeute: 48,1 % der Theorie,
Spektren: IR (Methylenchlorid): 1650 $cm^{-1}$ (Lactam-CO)
MS: $M^+$ 520 m/e

Beispiel 36

N-[3-(7,8-Dimethoxy-1,3,4,5-tetrahydro-2H-3-benzazepin-2-on-3-yl)-propyl]-N-[2-(4-amino-3,5-dichlorphenylamino)-ethyl]-methylamin

Hergestellt aus N-[3-(7,8-Dimethoxy-1,3,4,5-tetrahydro-2H-3-benzazepin-2-on-3-yl)-propyl]-2-(4-amino-3,5-dichlorphenyl-amino)-ethylamin, Paraformaldehyd und Natriumcyanborhydrid analog Beispiel 5.

Ausbeute: 75 % der Theorie,

Spektren: IR (Methylenchlorid): 1650 $cm^{-1}$ (Lactam-CO)

MS: $M^+$ 494/496 m/e

Beispiel 37

N-[3-(7,8-Dimethoxy-1,3,4,5-tetrahydro-2H-3-benzazepin-2-on-3-yl)-propyl]-N-[2-(N-(4-dimethylamino-3,5-dichlorphenyl)-N-methyl)-aminoethyl]-methylamin

Hergestellt aus N-[3-(7,8-Dimethoxy-1,3,4,5-tetrahydro-2H-3-benzazepin-2-on-3-yl)-propyl]-2-(4-amino-3,5-dichlorphenyl-amino)-ethylamin, Paraformaldehyd und Natriumcyanborhydrid analog Beispiel 5.

Ausbeute: 69 % der Theorie,

Spektren: IR (Methylenchlorid): 1650 $cm^{-1}$ (Lactam-CO)

MS: $M^+$ 536/538/540 m/e ($Cl_2$)

Beispiel 38

N-[3-(7,8-Dimethoxy-1,3,4,5-tetrahydro-2H-3-benzazepin-2-on-3-yl)-propyl]-N-[3-(N-(4-dimethylamino-3,5-dichlorphenyl)-N-methyl-amino)-propyl]-methylamin

Hergestellt aus N-[3-(7,8-Dimethoxy-1,3,4,5-tetrahydro-2H-3-benzazepin-2-on-3-yl)-propyl]-3-(4-amino-3,5-dichlorphenyl-amino)-propylamin, Paraformaldehyd und Natriumcyanborhydrid analog Beispiel 5.
Ausbeute: 74 % der Theorie,
Spektren: IR (Methylenchlorid): 1650 $cm^{-1}$ (Lactam-CO)
MS: $M^+$ 550/552/554 m/e ($Cl_2$)

Beispiel 39

N-[3-(7,8-Dimethoxy-1,3,4,5-tetrahydro-2H-3-benzazepin-2-on-3-yl)-propyl]-N-[3-(4-amino-3,5-dichlorphenylamino)-propyl]-methylamin

Hergestellt aus N-[3-(7,8-Dimethoxy-1,3,4,5-tetrahydro-2H-3-benzazepin-2-on-3-yl)-propyl]-3-(4-amino-3,5-dichlorphenyl-amino)-propylamin, Paraformaldehyd und Natriumcyanborhydrid analog Beispiel 5.
Ausbeute: 36 % der Theorie,
Spektren: IR (Methylenchlorid): 1650 $cm^{-1}$ (Lactam-CO)
MS: $M^+$ 508/510/512 m/e ($Cl_2$)

Beispiel 40

N-[3-(7,8-Dimethoxy-1,3,4,5-tetrahydro-2H-3-benzazepin-2-on-3-yl)-propyl]-N-[2-(4-amino-3,5-dibromphenylamino)-ethyl]-methylamin

Hergestellt aus N-[3-(7,8-Dimethoxy-1,3,4,5-tetrahydro-2H-3-benzazepin-2-on-3-yl)-propyl]-2-(4-amino-3,5-dibromphenylamino)-ethylamin, Paraformaldehyd und Natriumcyanborhydrid analog Beispiel 5.

Ausbeute: 77 % der Theorie,

Spektren: IR (Methylenchlorid): 1650 $cm^{-1}$ (Lactam-CO)

MS: $M^+$ 582/584/586 m/e ($Br_2$)

Beispiel 41

N-[3-(7,8-Dimethoxy-1,3,4,5-tetrahydro-2H-3-benzazepin-2-on-3-yl)-propyl]-N-[2-(N-(4-dimethylaminophenyl)-N-methylamino)-ethyl]-methylamin-hydrochlorid

Hergestellt aus N-[3-(7,8-Dimethoxy-1,3,4,5-tetrahydro-2H-3-benzazepin-2-on-3-yl)-propyl]-2-(4-dimethylaminophenylamino)-ethylamin, Paraformaldehyd und Natriumcyanborhydrid analog Beispiel 5.

Ausbeute: 68 % der Theorie,

Schmelzpunkt: 176-179°C (Zers.)

Beispiel 42

N-[3-(7,8-Dimethoxy-1,3,4,5-tetrahydro-2H-3-benzazepin-2-on-3-yl)-propyl]-N-[3-(N-(4-dimethylaminophenyl)-N-methyl-amino)-propyl]-methylamin-hydrochlorid

Hergestellt aus N-[3-(7,8-Dimethoxy-1,3,4,5-tetrahydro-2H-3-benzazepin-2-on-3-yl)-propyl]-3-(4-dimethylaminophenyl-amino)-propylamin, Paraformaldehyd und Natriumcyanborhydrid analog Beispiel 5.

Ausbeute: 64 % der Theorie,

Schmelzpunkt: 210-215°C (Zers.)

Beispiel 43

N-[3-(7,8-Dimethoxy-1,3,4,5-tetrahydro-2H-3-benzazepin-2-on-3-yl)-propyl]-N-[3-(4-amino-3,5-dibromphenylamino)-propyl]-methylamin

Hergestellt aus N-[3-(7,8-Dimethoxy-1,3,4,5-tetrahydro-2H-3-benzazepin-2-on-3-yl)-propyl]-3-(4-amino-3,5-dibromphenyl-amino)-propylamin, Paraformaldehyd und Natriumcyanborhydrid analog Beispiel 5.

Ausbeute: 62,5 % der Theorie,

Spektren: IR (Methylenchlorid): 1650 $cm^{-1}$ (Lactam-CO)

MS: $M^+$ 596/598/600 m/e ($Br_2$)

Beispiel 44

N-[3-(7,8-Dimethoxy-1,3,4,5-tetrahydro-2H-3-benzazepin-2-on-3-yl)-propyl]-N-[2-(N-(4-amino-3,5-dibromphenyl)-N-methyl-amino)-ethyl]-methylamin

Hergestellt aus N-[3-(7,8-Dimethoxy-1,3,4,5-tetrahydro-2H-3-benzazepin-2-on-3-yl)-propyl]-2-(4-amino-3,5-dibromphenyl-amino)-ethylamin, Paraformaldehyd und Natriumcycanborhydrid analog Beispiel 5.
Ausbeute: 50 % der Theorie,
Spektren: IR (Methylenchlorid): 1650 $cm^{-1}$ (Lactam-CO)
MS: $M^+$ 596/598/600 m/e ($Br_2$)

Beispiel 45

N-[3-(7,8-Dimethoxy-1,3,4,5-tetrahydro-2H-3-benzazepin-2-on-3-yl)-propyl]-N-[3-(N-(4-amino-3,5-dibromphenyl)-N-methyl-amino)-propyl]-methylamin-dihydrochlorid

Hergestellt aus N-[3-(7,8-Dimethoxy-1,3,4,5-tetrahydro-2H-3-benzazepin-2-on-3-yl)-propyl]-3-(4-amino-3,5-dibromphenyl-amino)-propylamin, Paraformaldehyd und Natriumcyanborhydrid analog Beispiel 5.
Ausbeute: 43 % der Theorie,
Schmelzpunkt: 204-205°C (Zers.)

Beispiel 46

N-[3-(7,8-Dimethoxy-1,3,4,5-tetrahydro-2H-3-benzazepin-2-on-3-yl)-propyl]-N-[2-(N-(4-amino-3,5-dichlorphenyl)-N-methyl-amino)-ethyl]-methylamin

Hergestellt aus N-[3-(7,8-Dimethoxy-1,3,4,5-tetrahydro-2H-3-benzazepin-2-on-3-yl)-propyl]-2-(4-amino-3,5-dichlorphenyl-amino)-ethylamin, Paraformaldehyd und Natriumcyanborhydrid analog Beispiel 5.

Ausbeute: 85 % der Theorie,

Spektren: IR (Methylenchlorid): 1650 $cm^{-1}$ (Lactam-CO)

MS: $M^+$ 508/510/512 m/e ($Cl_2$)

Beispiel 47

N-[3-(7,8-Dimethoxy-1,3,4,5-tetrahydro-2H-3-benzazepin-2-on-3-yl)-propyl]-N-[3-(N-phenyl-N-methyl-amino)-propyl]-methylamin

Hergestellt aus N-[3-(7,8-Dimethoxy-1,3,4,5-tetrahydro-2H-3-benzazepin-2-on-3-yl)-propyl]-3-phenylamino-propylamin, Paraformaldehyd und Natriumcyanborhydrid analog Beispiel 5.

Ausbeute: 72 % der Theorie,

Spektren: IR (Methylenchlorid): 1650 $cm^{-1}$ (Lactam-CO)

MS: $M^+$ 439 m/e

Beispiel 48

N-[3-(7,8-Dimethoxy-1,3,4,5-tetrahydro-2H-3-benzazepin-2-on-3-yl)-propyl]-N-[3-(N-(3,4-dimethoxyphenyl)-N-methyl-amino)-propyl]-methylamin

---

Hergestellt aus N-[3-(7,8-Dimethoxy-1,3,4,5-tetrahydro-2H-3-benzazepin-2-on-3-yl)-propyl]-3-(3,4-dimethoxyphenylamino)-propylamin, Paraformaldehyd und Natriumcyanborhydrid analog Beispiel 5.

Ausbeute: 74 % der Theorie,

Spektren: IR (Methylenchlorid): 1650 $cm^{-1}$ (Lactam-CO)

MS: M 499/me

Beispiel 49

N-[3-(7,8-Dimethoxy-1,3,4,5-tetrahydro-2H-3-benzazepin-2-on-3-yl)-propyl]-N-[2-(3,4-dimethoxyphenylamino)-ethyl]-methylamin

---

Hergestellt aus N-[3-(7,8-Dimethoxy-1,3,4,5-tetrahydro-2H-3-benzazepin-2-on-3-yl)-propyl]-2-(3,4-dimethoxyphenylamino)-ethylamin, Paraformaldehyd und Natriumcyanborhydrid analog Beispiel 5.

Ausbeute: 72 % der Theorie,

Spektren: IR (Methylenchlorid): 1650 $cm^{-1}$ (Lactam-CO)

MS: $M^+$ 471 m/e

Beispiel 50

N-[3-(7,8-Dimethoxy-1,3,4,5-tetrahydro-2H-3-benzazepin-2-on-3-yl)-propyl]-N-[3-(3,4-dimethoxyphenylamino)-propyl]-methyl-amin

Hergestellt aus N-[3-(7,8-Dimethoxy-1,3,4,5-tetrahydro-2H-3-benzazepin-2-on-3-yl)-propyl]-3-(3,4-dimethoxyphenylamino)-propylamin, Paraformaldehyd und Natriumcyanborhydrid analog Beispiel 5.
Ausbeute: 45 % der Theorie,
Spektren: IR (Methylenchlorid): 1650 $cm^{-1}$ (Lactam-CO)
MS: $M^+$ 485 m/e

Beispiel 51

N-[3-(7,8-Dimethoxy-1,3,4,5-tetrahydro-2H-3-benzazepin-2-on-3-yl)-propyl]-N-[3-(4-aminophenylthio)-propyl]-methylamin

Hergestellt aus N-[3-(7,8-Dimethoxy-1,3,4,5-tetrahydro-2H-3-benzazepin-2-on-3-yl)-propyl]-N-(3-chlorpropyl)-methylamin und 4-Aminothiophenol analog Beispiel 8.
Ausbeute: 15 % der Theorie,
Spektren: MS: $M^+$ 457 m/e

Beispiel 52

N-[3-(7,8-Dimethoxy-1,3,4,5-tetrahydro-2H-3-benzazepin-2-on-3-yl)-propyl]-N-[2-(4-amino-3,5-dibromphenyloxy)-ethyl]-methylamin-hydrochlorid

Hergestellt aus 3-(7,8-Dimethoxy-1,3,4,5-tetrahydro-2H-3-

benzazepin-2-on-3-yl)-l-chlorpropan und N-[2-(4-amino-3,5-dibromphenyloxy)-ethyl]-methylamin analog Beispiel 1.
Ausbeute: 44,3 % der Theorie,
Schmelzpunkt: 161-163°C

Beispiel 53

N-[3-(7,8-Dimethoxy-1,3,4,5-tetrahydro-2H-3-benzazepin-2-on-3-yl)-propyl]-N-(2-phenyloxyethyl)-methylamin-hydrochlorid

Hergestellt aus 3-(7,8-Dimethoxy-1,3,4,5-tetrahydro-2H-3-benzazepin-2-on-3-yl)-l-chlorpropan und N-(2-Phenyloxy-ethyl)-methylamin analog Beispiel 1.
Ausbeute: 47,7 % der Theorie,
Schmelzpunkt: 186-188°C

Beispiel 54

N-[3-(7,8-Dimethoxy-1,3,4,5-tetrahydro-2H-3-benzazepin-2-on-3-yl)-propyl]-N-[2-(3,4-dimethoxyphenyloxy)-ethyl]-methyl-amin-hydrochlorid

Hergestellt aus N-[3-(7,8-Dimethoxy-1,3,4,5-tetrahydro-2H-3-benzazepin-2-on-3-yl)-propyl]-methylamin und 2-(3,4-Di-methoxyphenyloxy)-ethylchlorid analog Beispiel 3.
Ausbeute: 11,6 % der Theorie,
Schmelzpunkt: 214-216°C

Beispiel 55

N-[3-(7,8-Dimethoxy-1,3,4,5-tetrahydro-2H-3-benzazepin-1,2-dion-3-yl)-propyl]-N-[2-(3,4-dichlorphenyloxy)-ethyl]-methylamin-hydrochlorid

5,0 g (0,00965 Mol) N-[3-(7,8-Dimethoxy-1,3,4,5-tetrahydro-2H-3-benzazepin-2-on-3-yl)-propyl]-N-[2-(3,4-dichlorphenyloxy)-ethyl]-methylamin-hydrochlorid werden mit 1,664 g (0,015 Mol) Selendioxid und 1 g Celite in einem Gemisch aus 100 ml Dioxan und 2 ml Wasser 1,5 Stunden lang zum Sieden erhitzt. Man engt im Vakuum zur Trockne ein, verteilt den Rückstand zwischen Essigester und 2n Natronlauge, filtriert vom Unlöslichen ab, trennt die organische Phase ab, wäscht sie mit Wasser, trocknet sie über Natriumsulfat und engt wieder zur Trockne ein. Das erhaltene Rohprodukt wird durch Chromatographie an Kieselgel (Elutionsmittel: Methylenchlorid/Methanol = 19:1) gereinigt. Die so erhaltene Base wird auf üblichem Wege in ihr Hydrochlorid überführt.
Ausbeute: 2,6 g (50,7 % der Theorie),
Schmelzpunkt: 230-235°C (Zers.).

Beispiel 56

N-[3-(7,8-Dimethoxy-1,3,4,5-tetrahydro-2H-1,3-benzdiazepin-2-on-3-yl)-propyl]-N-[3-(3,4-dimethoxyphenylthio)-propyl]-methylamin

2,5 g (0,0052 Mol) N-[3-(2-(2-Amino-4,5-dimethoxy-phenyl)-äthylamino)-propyl]-N-[3-(3,4-dimethoxyphenylthio)-propyl]-methylamin werden in 50 ml Acetonitril gelöst. Diese Lösung versetzt man mit 1,95 g (0,012 Mol) Carbonyldiimidazol und kocht 3 Stunden am Rückfluß. Man engt ein und kristallisiert den festen Rückstand aus 50 ml absolutem Ethanol um.

Ausbeute: 1,6 g (61,5 % der Theorie),
Schmelzpunkt: 124-126°C
IR-Spektrum (Methylenchlorid): 1665 $cm^{-1}$ (Carbonyl)

Beispiel 57

N-[3-(7,8-Dimethoxy-1,3,4,5-tetrahydro-2H-3-benzazepin-2-on-3-yl)-propyl]-N-[2-(4-dimethylamino-phenylthio)-ethyl]-methylamin

2,2 g (0,00496 Mol) N-[3-(7,8-Dimethoxy-1,3,4,5-tetrahydro-2H-3-benzazepin-2-on-3-yl)-propyl]-N-[2-(4-amino-phenylthio)-ethyl]-methylamin werden in 2 ml 90%iger Ameisensäure gelöst. Es werden 2 ml 35%ige Formalinlösung hinzugefügt und 2 Stunden unter Rückfluß gekocht. Man engt ein, versetzt den Rückstand mit Eis/Wasser, stellt mit 10 n Natronlauge alkalisch und isoliert mit Methylenchlorid die Base. Die organische Phase wird mit Wasser gewaschen über Natriumsulfat getrocknet und eingeengt. Das erhaltene Rohprodukt wird durch Chromatographie an Kieselgel (Elutionsmittel: Methylenchlorid/Methanol = 10:1) gereinigt.
Ausbeute: 0,3 g Öl (12,8 % der Theorie),
IR-Spektrum (Methylenchlorid): 1650 $cm^{-1}$ (Carbonyl)
MS: $M^+$ 471 m/e

Beispiel 58

N-[3-(7,8-Dimethoxy-1,3,4,5-tetrahydro-2H-1,3-benzdiazepin-2-on-3-yl)-propyl]-N-[3-(phenylthio)-propyl]-methylamin

Hergestellt aus N-[3-(2-(2-Amino-4,5-dimethoxy-phenyl)-ethylamino)-propyl]-N-[3-(phenylthio)-propyl]-methylamin und Carbonyldiimidazol in Acetonitril analog Beispiel 56.

Ausbeute: 40,4 % der Theorie,
Schmelzpunkt: 107-110°C
IR-Spektrum (Methylenchlorid): 1665 $cm^{-1}$ (Carbonyl)

Beispiel 59

N-[3-(7,8-Dimethoxy-1,3,4,5-tetrahydro-2H-3-benzazepin-2-on-3-yl)-propyl]-N-[2-(4-amino-3,5-dibromphenylthio)-äthyl]-methylamin

2,3 g (0,006 Mol) N-[3-(7,8-Dimethoxy-1,3,4,5-tetrahydro-2H-3-benzazepin-2-on-3-yl)-propyl]-N-[2-(4-amino-phenylthio)-ethyl]-methylamin werden in 100 ml 95%iger Essigsäure gelöst. Hierzu werden unter Rühren 1,92 g (0,012 Mol) Brom, gelöst in 10 ml Eisessig, zugetropft. Die Lösung färbt sich schwarz-blau. Nach beendigter Zugabe wird 30 Minuten weiter gerührt. Das Lösungsmittel entfernt man im Vakuum und versetzt den Rückstand mit Wasser und 10 n Natronlauge. Man schüttelt mit Methylenchlorid aus, wäscht die organische Phase mit Wasser, trocknet über Natriumsulfat und engt ein. Der Rückstand wird durch Chromatographie über Kieselgel (Elutionsmittel: Methylenchlorid/Methanol = 10:1) gereinigt. Ausbeute: 0,38 g Harz (10,5 % der Theorie).

Beispiel 60

N-[3-(7,8-Dimethoxy-1,3,4,5-tetrahydro-2H-3-benzazepin-2-on-3-yl)-propyl]-N-[2-(4-amino-3,5-dibrom-phenylsulfinyl)-ethyl]-methylamin

Hergestellt aus N-[3-(7,8-Dimethoxy-1,3,4,5-tetrahydro-2H-3-benzazepin-2-on-3-yl)-propyl]-N-[2-(4-amino-3,5-dibrompheny-

nylthio)-ethyl]-methylamin und 30%igem Wasserstoffperoxid (in Eisessig) analog Beispiel 4.
Ausbeute: 62,5 % der Theorie,
IR-Spektrum (Methylenchlorid): 1655 $cm^{-1}$ (Carbonyl)

## Beispiel 61

N-[3-(7,8-Dimethoxy-1,3,4,5-tetrahydro-2H-3-benzazepin-2-on-3-yl)-propyl]-N-[2-(4-amino-3,5-dibromphenyl-sulfonyl)-ethyl]-methylamin

Hergestellt aus N-[3-(7,8-Dimethoxy-1,3,4,5-tetrahydro-2H-3-benzazepin-2-on-3-yl)-propyl]-N-[2-(4-amino-3,5-dibromphenyl-thio)-ethyl]-methylamin und 30%igem Wasserstoffperoxid analog Beispiel 7.
Ausbeute: 52,6 % der Theorie,
IR-Spektrum (Methylenchlorid): 1655 $cm^{-1}$ (Carbonyl)

## Beispiel 62

N-[3-(7,8-Dimethoxy-1,3,4,5-tetrahydro-2H-3-benzazepin-2-on-3-yl)-propyl]-N-[2-(4-dimethylaminophenyloxy)-ethyl]-methylamin-dihydrochlorid

Hergestellt aus 3-(7,8-Dimethoxy-1,3,4,5-tetrahydro-2H-3-benzazepin-2-on-3-yl)-1-chlorpropan, N-[2-(4-Dimethylamino-phenyloxy)-ethyl]-methylamin und Triethylamin analog Beispiel 6.
Ausbeute: 8,4 % der Theorie,
Schmelzpunkt: ab 83°C (Zers.)

| | | | | | | | | |
|---|---|---|---|---|---|---|---|---|
| Ber. | C | 59,09 | H | 7,44 | Cl | 13,42 | N | 7,95 |
| Gef. | | 59,43 | | 7,80 | | 13,36 | | 7,54 |

Beispiel 63

N-[3-(7,8-Dimethoxy-1,3,4,5-tetrahydro-2H-3-benzazepin-2-on-3-yl)-propyl]-N-[3-(4-dimethylaminophenyloxy)-propyl]-methylamin-dihydrochlorid

Hergestellt aus 3-(7,8-Dimethoxy-1,3,4,5-tetrahydro-2H-3-benzazepin-2-on-3-yl)-1-chlorpropan, N-[3-(4-Dimethylaminophenyloxy)-propyl]-methylamin und Triethylamin analog Beispiel 6.
Ausbeute: 19,4 % der Theorie,
Schmelzpunkt: ab 86 °C (Zers.)

| | | | | | | | | |
|---|---|---|---|---|---|---|---|---|
| Ber. | C | 59,77 | H | 7,62 | Cl | 13,07 | N | 7,75 |
| Gef. | | 59,77 | | 7,67 | | 13,49 | | 7,78 |

MS: $M^+$ 469 m/e

Beispiel 64

N-[3-(7,8-Dimethoxy-1,3,4,5-tetrahydro-2H-3-benzazepin-2-on-3-yl)-propyl]-N-[3-(4-amino-3,5-dibrom-phenyloxy)-propyl]-methylamin-hydrochlorid

Hergestellt aus 3-(7,8-Dimethoxy-1,3,4,5-tetrahydro-2H-3-benzazepin-2-on-3-yl)-1-chlorpropan, N-[3-(4-Amino-3,5-dibrom-phenyloxy)-propyl]-methylamin und Triethylamin analog Beispiel 6.
Ausbeute: 20,2 % der Theorie,

| | | | | | | | | | | |
|---|---|---|---|---|---|---|---|---|---|---|
| Ber. | C | 47,37 | H | 5,09 | Cl | 5,59 | Br | 25,22 | N | 6,63 |
| Gef. | | 47,19 | | 5,06 | | 5,17 | | 24,83 | | 6,24 |

MS: $M^+$ 597 m/e

Beispiel 65

N-[3-(7,8-Dimethoxy-1,3,4,5-tetrahydro-2H-3-benzazepin-1,2-dion-3-yl)-propyl]-N-[3-(phenylthio)-propyl]-methylamin

Hergestellt aus N-[3-(7,8-Dimethoxy-1,3,4,5-tetrahydro-2H-3-benzazepin-2-on-3-yl)-propyl]-N-[3-(phenylthio)-propyl]-methylamin und Selendioxyd analog Beispiel 55.
Ausbeute: 39,06 % der Theorie,
Schmelzpunkt: 77-79°C
IR-Spektrum (Methylenchlorid): 1660 $cm^{-1}$ (Carbonyl)

Beispiel 66

N-[3-(7,8-Dimethoxy-1,3,4,5-tetrahydro-2H-3-benzazepin-1,2-dion-3-yl)-propyl]-N-[3-(phenylsulfonyl)-propyl]-methylamin

Hergestellt aus N-[3-(7,8-Dimethoxy-1,3,4,5-tetrahydro-2H-3-benzazepin-2-on-3-yl)-propyl]-N-[3-(phenylthio)-propyl]-methylamin und Selendioxyd analog Beispiel 55.
Ausbeute: 59,2 % der Theorie,
IR-Spektrum (Methylenchlorid): 1665 $cm^{-1}$ (Carbonyl)

Beispiel 67

N-[3-(7,8-Dimethoxy-1,3,4,5-tetrahydro-2H-1,3-benzdiazepin-2-on-3-yl)-propyl]-N-[3-(phenylsulfonyl)-propyl]-methylamin

Hergestellt aus N-[3-(7,8-Dimethoxy-1,3,4,5-tetrahydro-2H-1,3-benzdiazepin-2-on-3-yl)-propyl]-N-[3-(phenylthio)-propyl]-methylamin und 30%igem Wasserstoffperoxid (in Eisessig) analog Beispiel 7.

Ausbeute: 51,8 % der Theorie,
Schmelzpunkt: 118-121°C
IR-Spektrum (Methylenchlorid/KBr): 1670 $cm^{-1}$ (Carbonyl)

Beispiel 68

N-[3-(7,8-Dimethoxy-1,3,4,5-tetrahydro-2H-3-benzazepin-2-on-3-yl)-propyl]-N-[2-(3,4-dimethoxyphenyl)-ethyl]-propylamin-hydrochlorid

Hergestellt aus N-[3-(7,8-Dimethoxy-1,3,4,5-tetrahydro-2H-3-benzazepin-2-on-3-yl)-propyl]-N-2-(3,4-dimethoxyphenyl)-ethylamin, Propionaldehyd und Natriumcyanborhydrid analog Beispiel 5.
Ausbeute: 45,28 % der Theorie,
Schmelzpunkt: 56-70°C

Beispiel 69

N-[3-(7,8-Dimethoxy-1,3,4,5-tetrahydro-2H-3-benzazepin-1,2-dion-3-yl)-propyl]-N-[3-(3,4-dimethoxy-phenylthio)-propyl]-methylamin

Hergestellt aus N-[3-(7,8-Dimethoxy-1,3,4,5-tetrahydro-2H-3-benzazepin-2-on-3-yl)-propyl]-N-[3-(3,4-dimethoxy-phenylthio)-propyl]-methylamin und Selendioxyd analog Beispiel 55.
Ausbeute: 25 % der Theorie,
IR-Spektrum (Methylenchlorid): 1660 $cm^{-1}$ (Carbonyl)

Beispiel 70

N-[3-(7,8-Dimethoxy-1,3,4,5-tetrahydro-2H-3-benzazepin-1,2-dion-3-yl)-propyl]-N-[3-(3,4-dimethoxy-phenyl-sulfinyl)-propyl]-methylamin

Hergestellt aus N-[3-(7,8-Dimethoxy-1,3,4,5-tetrahydro-2H-3-benzazepin-2-on-3-yl)-propyl]-N-[3-(3,4-dimethoxy-phenyl-sulfinyl)-propyl]-methylamin und Selendioxyd analog Beispiel 55.
Ausbeute: 41,6 % der Theorie,
IR-Spektrum (Methylenchlorid): 1660 $cm^{-1}$ (Carbonyl)

Beispiel 71

N-[3-(7,8-Dimethoxy-1,3,4,5-tetrahydro-2H-3-benzazepin-1,2-dion-3-yl)-propyl]-N-[3-(3,4-dimethoxy-phenylsulfonyl)-propyl]-methylamin

Hergestellt aus N-[3-(7,8-Dimethoxy-1,3,4,5-tetrahydro-2H-3-benzazepin-2-on-3-yl)-propyl]-N-[3-(3,4-dimethoxy-phenylsulfonyl)-propyl]-methylamin und Selendioxyd analog Beispiel 55.
Ausbeute: 50 % der Theorie,
IR-Spektrum (Methylenchlorid): 1660 $cm^{-1}$ (Carbonyl)

Beispiel 72

N-[3-(7,8-Dimethoxy-1,3,4,5-tetrahydro-2H-3-benzazepin-1,2-dion-3-yl)-propyl]-N-[3-(4-amino-3,5-dibrom-phenylthio)-propyl]-methylamin

Hergestellt aus 3-(7,8-Dimethoxy-1,3,4,5-tetrahydro-2H-3-

benzazepin-1,2-dion-3-yl)-propylchlorid und N-[3-(4-Amino-3,5-dibrom-phenylthio)-propyl]-methylamin analog Beispiel 1.
Ausbeute: 60,6 % der Theorie,
Schmelzpunkt: 118-125°C
IR-Spektrum (Methylenchlorid): 1660 $cm^{-1}$ (Carbonyl)

<u>Beispiel 73</u>

<u>N-[3-(7,8-Dimethoxy-1,3,4,5-tetrahydro-2H-3-benzazepin-1,2-dion-3-yl)-propyl]-2-(3,4-dimethoxyphenylamino)-ethylamin</u>

Hergestellt aus 3-(7,8-Dimethoxy-1,3,4,5-tetrahydro-2H-3-benzazepin-1,2-dion-3-yl)-propylchlorid und 2-(3,4-Dimethoxyphenylamino)-ethylamin analog Beispiel 6.
Ausbeute: 6 % der Theorie,
IR-Spektrum (Methylenchlorid): 1660 $cm^{-1}$ (Carbonyl)
MS: $M^+$ 471 m/e

<u>Beispiel 74</u>

<u>N-[3-(7,8-Dimethoxy-1,3,4,5-tetrahydro-2H-3-benzazepin-1,2-dion-3-yl)-propyl]-2-(4-amino-3,5-dichlorphenylamino)-ethylamin</u>

Hergestellt aus 3-(7,8-Dimethoxy-1,3,4,5-tetrahydro-2H-3-benzazepin-1,2-dion-3-yl)-propylchlorid und 2-(4-Amino-3,5-dichlorphenylamino)-ethylamin analog Beispiel 6.
Ausbeute: 38 % der Theorie,
IR-Spektrum (Methylenchlorid): 1645 $cm^{-1}$ (Carbonyl)
MS: $M^+$ 695/7/9 m/e

Beispiel 75

N-[3-(7,8-Dimethoxy-1,3,4,5-tetrahydro-2H-3-benzazepin-2-on-3-yl)-propyl]-N-[2-(3,5-dichlor-4-methoxy-phenyloxy)-ethyl]-methylamin-hydrochlorid

Hergestellt aus 3-(7,8-Dimethoxy-1,3,4,5-tetrahydro-2H-3-benzazepin-2-on-3-yl)-1-chlorpropan, N-[2-(3,5-Dichlor-4-methoxy-phenyloxy)-ethyl]-methylamin und Triethylamin analog Beispiel 6.
Ausbeute: 23,9 % der Theorie,
Schmelzpunkt: ab 160°C (Zers.)

| | | | | | | | | |
|---|---|---|---|---|---|---|---|---|
| Ber.: | C | 54,80 | H | 6,07 | Cl | 19,41 | N | 5,11 |
| Gef.: | | 54,70 | | 6,01 | | 19,33 | | 5,08 |

Beispiel 76

N-[3-(7,8-Dimethoxy-1,3,4,5-tetrahydro-2H-3-benzazepin-1,2-dion-3-yl)-propyl]-N-[3-(4-amino-3,5-dibromphenyloxy)-propyl]-methylamin-hydrochlorid

Hergestellt aus 3-(7,8-Dimethoxy-1,3,4,5-tetrahydro-2H-3-benzazepin-1,2-dion-3-yl)-1-chlorpropan, N-[3-(4-Amino-3,5-dibrom-phenyloxy)-propyl]-methylamin und Triethylamin analog Beispiel 6.
Ausbeute: 27,8 % der Theorie,
Schmelzpunkt: ab 81°C (Zers.)

| | | | | | | | | | | |
|---|---|---|---|---|---|---|---|---|---|---|
| Ber.: | C | 46,21 | H | 4,96 | Cl | 5,46 | Br | 24,69 | N | 6,47 |
| Gef.: | | 46,22 | | 5,32 | | 5,53 | | 24,95 | | 6,76 |

Beispiel 77

N-[3-(7,8-Dimethoxy-1,3,4,5-tetrahydro-2H-3-benzazepin-1,2-dion-3-yl)-propyl]-N-[3-(3,4-dimethoxyphenyloxy)-propyl]-methylamin-hydrochlorid

---

Hergestellt aus 3-(7,8-Dimethoxy-1,3,4,5-tetrahydro-2H-3-benzazepin-1,2-dion-3-yl)-1-chlorpropan, N-[3-(3,4-Dimethoxyphenyloxy)-propyl]-methylamin und Triethylamin analog Beispiel 6.

Ausbeute: 44,1 % der Theorie,

Schmelzpunkt: ab 181°C (Zers.)

| | | | | | | | | |
|---|---|---|---|---|---|---|---|---|
| Ber.: | C | 60,38 | H | 6,94 | Cl | 6,60 | N | 5,22 |
| Gef.: | | 60,10 | | 7,17 | | 6,80 | | 5,12 |

Beispiel 78

N-[3-(7,8-Methylendioxy-1,3,4,5-tetrahydro-2H-1,3-benzdiazepin-2-on-3-yl)-propyl]-N-[3-(3,4-dimethoxy-phenylthio)-propyl]-methylamin

---

Hergestellt aus N-[3-(2-(2-Amino-4,5-methylendioxy-phenyl)-ethylamino)-propyl]-N-[3-(3,4-dimethoxyphenylthio)-propyl]-methylamin und Carbonyldiimidazol in Acetonitril analog Beispiel 56.

Ausbeute: 55,5 % der Theorie,

IR-Spektrum (Methylenchlorid): 3400 $cm^{-1}$ (NH)
1665 $cm^{-1}$ (CO)

Beispiel 79

N-[3-(7,8-Dimethoxy-1,3,4,5-tetrahydro-2H-3-benzazepin-1,2-dion-3-yl)-propyl]-N-[2-(N-(3,4-dimethoxy-phenyl)-methylamino)-ethyl]-methylamin

Hergestellt aus 3-(7,8-Dimethoxy-1,3,4,5-tetrahydro-2H-3-benzazepin-1,2-dion-3-yl)-1-chlorpropan und N-[2-[N-(3,4-Dimethoxy-phenyl)-methylamino]-ethyl]-methylamin analog Beispiel 6.
Ausbeute: 51 % der Theorie,
IR-Spektrum (Methylenchlorid): 1660 $cm^{-1}$ (Carbonyl)

Beispiel 80

N-[3-(7,8-Dimethoxy-1,3,4,5-tetrahydro-2H-3-benzazepin-2-on-3-yl)-propyl]-2-[N-(4-amino-3,5-dichlorphenyl)-methylamino]-ethylamin

Hergestellt aus 3-(7,8-Dimethoxy-1,3,4,5-tetrahydro-2H-3-benzazepin-2-on-3-yl)-1-chlorpropan, 2-[N-(4-Amino-3,5-dichlorphenyl)-methylamino]-ethylamin und Triethylamin analog Beispiel 6.
Ausbeute: 18 % der Theorie,
IR-Spektrum (Methylenchlorid): 1645 $cm^{-1}$ (Carbonyl)

Beispiel 81

N-[3-(7,8-Dimethoxy-1,3,4,5-tetrahydro-2H-3-benzazepin-2-on 3-yl)-propyl]-2-[N-(3,4-dimethoxyphenyl-methylamino]-ethylamin

---

Hergestellt aus 3-(7,8-Dimethoxy-1,3,4,5-tetrahydro-2H-3-benzazepin-2-on-3-yl)-1-chlorpropan und 2-[N-(3,4-Dimethoxyphenyl)-methylamino]-ethylamin analog Beispiel 6.
Ausbeute: 6,6 % der Theorie,
IR-Spektrum (Methylenchlorid): 1650 $cm^{-1}$ (Carbonyl)

Beispiel 82

N-[3-(7,8-Dimethoxy-1-benzyl-1,3,4,5-tetrahydro-2H-1,3-benzdiazepin-2-on-3-yl)-propyl]-2-(4-amino-3,5-dichlor-phenylamino)-ethylamin

---

Hergestellt aus 3-(7,8-Dimethoxy-1-benzyl-1,3,4,5-tetrahydro-2H-1,3-benzdiazepin-2-on-3-yl)-1-chlorpropan und 2-(4-Amino-3,5-dichlor-phenylamino)-ethylamin analog Beispiel 6.
Ausbeute: 51 % der Theorie,
IR-Spektrum (Methylenchlorid): 1615 $cm^{-1}$ (Carbonyl)
UV-Spektrum (Ethanol): 245 nm (0,16)
280 nm (0,04)
330 nm (0,03)

Beispiel 83

N-[3-(7,8-Dimethoxy-1-benzyl-1,3,4,5-tetrahydro-1H-1,3-benzdiazepin-2-on-3-yl)-propyl]-2-(3,4-dimethoxy-phenylamino)-ethylamin

Hergestellt aus 3-(7,8-Dimethoxy-1-benzyl-1,3,4,5-tetrahydro-1H-1,3-benzdiazepin-2-on-3-yl)-1-chlorpropan und 2-(3,4-Dimethoxy-phenylamino)-ethylamin analog Beispiel 6.
Ausbeute: 39 % der Theorie,
MS: $M^+$ 548 m/e

Beispiel 84

N-[3-(7,8-Dimethoxy-1-benzyl-1,3,4,5-tetrahydro-2H-1,3-benzdiazepin-2-on-3-yl)-propyl]-N-[2-(N-methyl-3,4-dimethoxy-phenylamino)-ethyl]-methylamin

Hergestellt aus 3-(7,8-Dimethoxy-1-benzyl-1,3,4,5-tetrahydro-2H-1,3-benzdiazepin-2-on-3-yl)-1-chlorpropan und N-[2-(N-Methyl-3,4-dimethoxy-phenylamino)-ethyl]-methylamin analog Beispiel 6.
Ausbeute: 67 % der Theorie,
IR-Spektrum (Methylenchlorid): 1620 $cm^{-1}$ (Carbonyl)
MS: $M^+$ 576 m/e

Beispiel 85

N-[3-(7,8-Dimethoxy-1,3,4,5-tetrahydro-2H-1,3-benzdiazepin-2-on-3-yl)-propyl]-N-[2-(N-methyl-3,4-dimethoxy-phenylamino)-ethyl]-methylamin

Hergestellt aus N-[3-(2-(2-Amino-4,5-dimethoxyphenyl)-ethylamino)-propyl]-N-[2-(N-methyl-3,4-dimethoxy-phenylamino)-ethyl]-methylamin und Carbonyldiimidazol analog Beispiel 56.
Ausbeute: 48 % der Theorie,
Schmelzpunkt: 117-119°C
IR-Spektrum (Methylenchlorid): 1670 $cm^{-1}$ (Carbonyl)

Beispiel 86

N-[3-(7,8-Dimethoxy-1,3,4,5-tetrahydro-2H-1,3-benzdiazepin-2-on-3-yl)-propyl]-N-[2-(N-methyl-3,4-dimethoxy-phenylamino)-ethyl]-methylamin

Hergestellt aus N-[3-(7,8-Dimethoxy-1-benzyl-1,3,4,5-tetrahydro-2H-1,3-benzdiazepin-2-on-3-yl)-propyl]-N-[2-(N-methyl-3,4-dimethoxy-phenylamino)-ethyl]-methylamin durch Hydrogenolyse in Äthanol bei pH 2-3 in Gegenwart von Palladium/Kohle.
Ausbeute: 53 % der Theorie,
Schmelzpunkt: 117-119°C,
IR-Spektrum (Methylenchlorid): 2840 $cm^{-1}$ ($OCH_3$)
2800 $cm^{-1}$ (N-alkyl)
1670 $cm^{-1}$ (Carbonyl)

Analog den Beispielen 1 bis 8, 55 bis 57, 59 und 86 können folgende Verbindungen hergestellt werden:

N-[3-(7,8-Dimethoxy-1,3,4,5-tetrahydro-2H-3-benzazepin-1,2-dion-3-yl)-propyl]-3-phenylaminopropylamin,

N-[3-(7,8-Dimethoxy-1,3,4,5-tetrahydro-2H-1,3-benzdiazepin-2-on-3-yl)-propyl]-N-(2-phenylthioethyl)-methylamin,

N-[3-(7,8-Dimethoxy-1,3,4,5-tetrahydro-2H-3-benzazepin-1,2-dion-3-yl)-propyl]-N-(2-phenylthioethyl)-methylamin,

N-[3-(7,8-Dimethoxy-1,3,4,5-tetrahydro-2H-1,3-benzdiazepin-2-on-3-yl)-propyl]-N-(2-phenylaminoethyl)-methylamin,

N-[3-(7,8-Dimethoxy-1,3,4,5-tetrahydro-2H-3-benzazepin-1,2-dion-3-yl)-propyl]-N-(2-phenylaminoethyl)-methylamin,

N-[3-(7,8-Dimethoxy-1,3,4,5-tetrahydro-2H-1,3-benzdiazepin-2-on-3-yl)-propyl]-2-(3,4-dimethoxyphenylamino)-ethylamin,

N-[3-(7,8-Dimethoxy-1,3-dihydro-2H-3-benzazepin-2-on-3-yl)-propyl]-2-phenyloxyethylamin,

N-[3-(7,8-Dimethoxy-1,3-dihydro-2H-3-benzazepin-2-on-3-yl)-propyl]-2-phenyloxyethyl-methylamin,

N-[3-(7,8-Dimethoxy-1,3-dihydro-2H-3-benzazepin-2-on-3-yl)-propyl]-2-phenylamino-ethylamin,

N-[3-(7,8-Dimethoxy-1,3,4,5-tetrahydro-2H-3-benzazepin-1,2-dion-3-yl)-propyl]-N-(3-phenylaminopropyl)-methylamin,

N-[3-(7,8-Dimethoxy-1,3,4,5-tetrahydro-2H-1,3-benzodiazepin-2-on-3-yl)-propyl]-N-(3-phenylaminopropyl)-methylamin,

N-[3-(7,8-Dimethoxy-1,3,4,5-tetrahydro-2H-3-benzazepin-1,2-dion-3-yl)-propyl]-3-(3,4-dimethoxyphenylamino)-propylamin,

N-[3-(7,8-Dimethoxy-1,3,4,5-tetrahydro-2H-1,3-benzodiazepin-2-on-3-yl)-propyl]-3-(3,4-dimethoxyphenylamino)-propylamin,

N-[3-(7,8-Dimethoxy-1,3,4,5-tetrahydro-2H-3-benzazepin-1,2-dion-3-yl)-propyl]-N-[3-(N-methyl-N-phenylamino)-propyl]-methylamin,

N-[3-(7,8-Dimethoxy-1,3,4,5-tetrahydro-2H-1,3-benzodiazepin-2-on-3-yl)-propyl]-N-[3-(N-methyl-N-phenylamino)-propyl]-methylamin,

N-[3-(7,8-Dimethoxy-1,3,4,5-tetrahydro-2H-3-benzazepin-1,2-dion-3-yl)-propyl]-N-[3-(N-methyl-N-(3,4-dimethoxyphenyl)-amino)-propyl]-methylamin,

N-[3-(7,8-Dimethoxy-1,3,4,5-tetrahydro-2H-1,3-benzodiazepin-2-on-3-yl)-propyl]-N-[3-(N-methyl-N-(3,4-dimethoxyphenyl)-amino)-propyl]-methylamin

Beispiel I

Tabletten zu 10 mg N-[3-(7,8-Dimethoxy-1,3,4,5-tetrahydro-2H-3-benzazepin-2-on-3-yl)-propyl]-2-(3,4-dimethoxyphenyl-amino)-ethylamin-dihydrochlorid

Zusammensetzung:

1 Tablette enthält:

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel II

Dragées zu 5 mg N-[3-(7,8-Dimethoxy-1,3,4,5-tetrahydro-2H-3-benzazepin-2-on-3-yl)-propyl]-2-(3,4-dimethoxyphenylamino)-ethylamin-dihydrochlorid

1 Dragéekern enthält:

| | |
|---|---|
| Wirksubstanz | 5,0 mg |
| Maisstärke | 41,5 mg |

| | |
|---|---|
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Drageekerne mit einem Druchmesser von 6 mm gepreßt. Die so hergestellten Drageekerne werden auf bekannte Weise mit einer Schicht überzogen, die im wesentlichen aus Zucker und Talkum besteht. Die fertigen Dragées werden mit Wachs poliert.

Drageegewicht: 130 mg

Beispiel III

Ampullen zu 5 mg N-[3-(7,8-Dimethoxy-1,3,4,5-tetrahydro-2H-3-benzazepin-2-on-3-yl)-propyl]-2-(3,4-dimethoxyphenylamino)-ethylamin-dihydrochlorid

1 Ampulle enthält:

| | | |
|---|---|---|
| Wirksubstanz | | 5,0 mg |
| Sorbit | | 50,0 mg |
| Wasser für Injektionszwecke | ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg N-[3-(7,8-Dimethoxy-1,3,4,5-tetrahydro-2H-3-benzazepin-2-on-3-yl)-propyl]-2-(3,4-dimethoxyphenylamino)-ethylamin-dihydrochlorid

1 Zäpfchen enthält:

| | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Beispiel V

Tropfenlösung mit 10 mg N-[3-(7,8-Dimethoxy-1,3,4,5-tetrahydro-2H-3-benzazepin-2-on-3-yl)-propyl]-2-(3,4-dimethoxyphenylamino)-ethylamin-dihydrochlorid

100 ml Lösungen enthalten:

| | | |
|---|---|---|
| Wirksubstanz | | 0,2 g |
| Hydroxyäthylcellulose | | 0,15 g |
| Weinsäure | | 0,1 g |
| Sorbitlösung 70 % Trockensubstanz | | 30,0 g |
| Glycerin | | 10,0 g |
| Benzoesäure | | 0,15 g |
| Dest.Wasser | ad | 100 ml |

Herstellungsverfahren:

Dest.Wasser wird auf 70°C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

Patentansprüche

1. Neue Benzazepinderivate der allgemeinen Formel

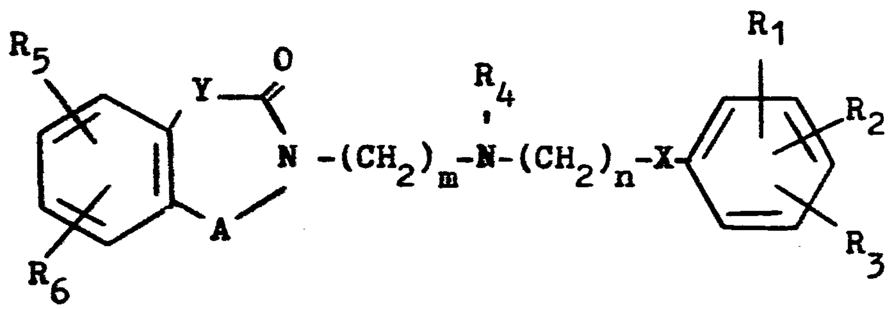

,(I)

in der

A eine $-CH_2CH_2-$ oder -CH=CH-Gruppe,

$R_1$ ein Wasserstoff-, Chlor- oder Bromatom, eine Alkyl-, Amino-, Alkylamino-, Dialkylamino-, Acylamino-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkyl-, Alkoxy- oder Phenylalkoxygruppe, wobei der Alkylteil jeweils 1 bis 3 Kohlenstoffatome enthalten kann, oder

$R_1$ und $R_2$ zusammen eine Alkylendioxigruppe mit 1 oder 2 Kohlenstoffatomen,

$R_3$ ein Wasserstoff-, Chlor- oder Bromatom oder eine Alkoxygruppe mit 1 bis 3 Kohlenstoffatomen,

$R_4$ ein Wasserstoffatom, eine Benzyl-, Alkyl- oder Alkenylgruppe, wobei der Alkylteil 1 bis 3 Kohlenstoffatome und der Alkenylteil 3 bis 5 Kohlenstoffatome enthalten kann,

$R_5$ ein Wasserstoff- oder Halogenatom, eine Alkyl- oder Alkoxygruppe mit jeweils 1 bis 3 Kohlenstoffatomen,

$R_6$ ein Wasserstoffatom, eine Alkyl- oder Alkoxygruppe mit jeweils 1 bis 3 Kohlenstoffatomen oder

$R_5$ und $R_6$ zusammen eine Alkylendioxigruppe mit 1 oder 2 Kohlenstoffatomen,

X eine gegebenenfalls durch eine Benzylgruppe oder eine Alkylgruppe mit 1 bis 3 Kohlenstoffatomen substituierte Iminogruppe, ein Sauerstoff- oder Schwefelatom, eine Sulfinyl- oder Sulfonylgruppe,

Y eine gegebenenfalls durch eine Benzylgruppe oder eine Alkylgruppe mit 1 bis 3 Kohlenstoffatomen substituierte Iminogruppe, eine Methylen- oder Carbonylgruppe,

n die Zahlen 2, 3 oder 4 und

m die Zahlen 2, 3 oder 4

bedeuten, und deren Säureadditionssalze mit anorganischen oder organischen Säuren.

2. Neue Benzazepinderivate der allgemeinen Formel I gemäß Anspruch 1, in der

A eine $-CH_2CH_2-$Gruppe,

Y eine $-CH_2-$, -CO- oder NH-Gruppe,

X ein Sauerstoff- oder Schwefelatom, eine NH-, $NCH_3-$, SO- oder $SO_2$-Gruppe,

n die Zahlen 2, 3 oder 4,

m die Zahl 3,

$R_1$ ein Wasserstoffatom, eine Methoxy-, Acetylamino-, Amino-, Methylamino- oder Dimethylaminogruppe,

$R_2$ ein Wasserstoff-, Chlor- oder Bromatom, eine Methoxygruppe oder $R_1$ und $R_2$ zusammen eine Methylendioxygruppe,

$R_3$ ein Wasserstoff-, Chlor- oder Bromatom oder eine Methoxygruppe,

$R_4$ ein Wasserstoffatom oder eine Methylgruppe,

$R_5$ und $R_6$ jeweils eine Methoxygruppe bedeuten, und deren Säureadditionssalze mit anorganischen oder organischen Säuren.

3. Neue Benzazepinderivate der allgemeinen Formel I gemäß Anspruch 1, in der

A eine $-CH_2CH_2-$Gruppe,

Y eine $-CH_2-$Gruppe,

X ein Sauerstoffatom, eine Imino-, Methylimino-, Sulfinyl- oder Sulfonylgruppe,

n die Zahlen 2 und 3,

m die Zahl 3,

$R_1$ ein Wasserstoffatom, eine Methoxy- oder Aminogruppe,

$R_2$ ein Wasserstoff-, Chlor- oder Bromatom, eine Methoxygruppe oder $R_1$ und $R_2$ zusammen eine Methylendioxygruppe,

$R_3$ ein Wasserstoff-, Chlor- oder Bromatom,

$R_4$ ein Wasserstoffatom oder eine Methylgruppe,

$R_5$ in 7-Stellung eine Methoxygruppe und

$R_6$ in 8-Stellung eine Methoxygruppe bedeuten, und deren Säureadditionssalze mit anorganischen oder organischen Säuren.

4. N-[3-(7,8-Dimethoxy-1,3,4,5-tetrahydro-2H-3-benzazepin-2-on-3-yl)-propyl]-N-(2-phenylamino)-ethyl-methylamin und dessen Säureadditionssalze.

5. N-[3-(7,8-Dimethoxy-1,3,4,5-tetrahydro-2H-3-benzazepin-2-on-3-yl)-propyl]-N-[2-(3,4-dimethoxyphenyloxy)-ethyl]-methylamin und dessen Säureadditionssalze.

6. N-[3-(7,8-Dimethoxy-1,3,4,5-tetrahydro-2H-3-benzazepin-2-on-3-yl)-propyl]-N-(3-phenylthio-propyl)-methylamin und dessen Säureadditionssalze.

7. Physiologisch verträgliche Säureadditionssalze der Verbindungen gemäß den Ansprüchen 1 bis 6 mit anorganischen oder organischen Säuren.

8. Arzneimittel, enthaltend eine Verbindung gemäß den Ansprüchen 1 bis 6 oder dessen physiologisch verträgliches Säureadditionssalz gemäß Anspruch 7 neben einem oder mehreren inerten Trägerstoffen und/oder Verdünnungsmitteln.

9. Verfahren zur Herstellung eines Arzneimittels gemäß Anspruch 8, dadurch gekennzeichnet, daß eine Verbindung gemäß den Ansprüchen 1 bis 6 oder dessen physiologisch verträgliches Säureadditionssalz gemäß Anspruch 7 in einen oder mehrere inerte übliche Trägerstoffe und/oder Verdünnungsmittel eingearbeitet wird.

 0161599

10. Verfahren zur Herstellung der Verbindungen gemäß Anspruch 1 bis 7, dadurch gekennzeichnet, daß

a) eine Verbindung der allgemeinen Formel

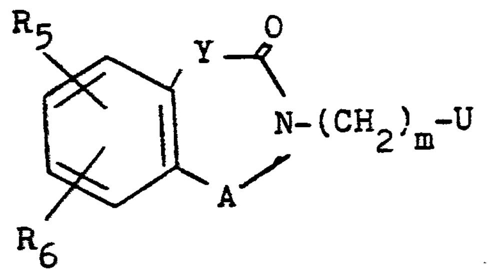

,(II)

mit einer Verbindung der allgemeinen Formel

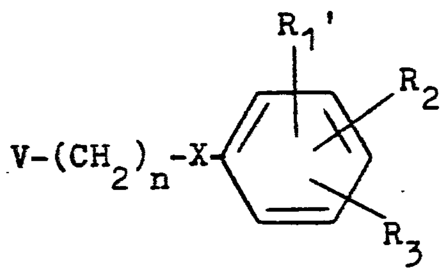

,(III)

in denen A, $R_2$, $R_3$, $R_5$, $R_6$, X, Y, m und n wie in den Ansprüchen 1 bis 6 definiert sind,

$R_1'$ eine durch einen Schutzrest geschützte Hydroxy-, Amino- oder Alkylaminogruppe darstellt oder die für $R_1$ in den Ansprüchen 1 bis 6 erwähnten Bedeutungen besitzt,

einer der Reste U oder V die $R_4$-NH-Gruppe darstellt, wobei $R_4$ wie in den Ansprüchen 1 bis 6 definiert ist, und

der andere der Reste U oder V eine nukleophile Austrittsgruppe bedeuten, umgesetzt und gegebenenfalls anschließend ein verwendeter Schutzrest abgespalten wird oder

b) zur Herstellung von Verbindungen der allgemeinen Formel I, in der $R_4$ eine Alkylgruppe und/oder $R_1$ eine Dialkylaminogruppe darstellt und X wie in den Ansprüchen 1 bis 6 definiert ist oder eine durch eine Alkylgruppe mit jeweils 1 bis 3 Kohlenstoffatomen substituierte Iminogruppe darstellen, eine Verbindung der allgemeinen Formel

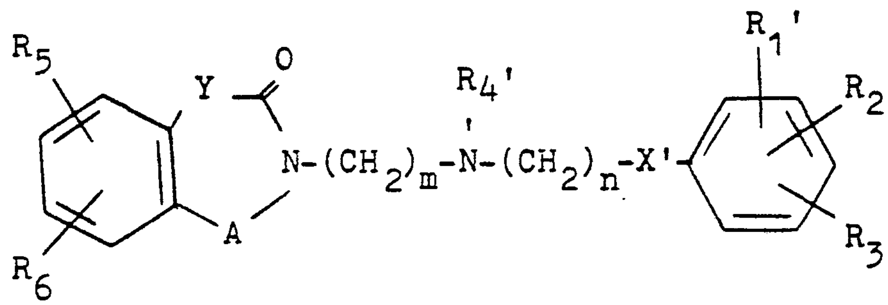 ,(IV)

in der
A, $R_2$, $R_3$, $R_5$, $R_6$, Y, m und n wie in den Ansprüchen 1 bis 6 definiert sind,

$R_1$' eine durch einen Schutzrest geschützte Hydroxy-, Amino- oder Alkylaminogruppe darstellt oder die für $R_1$ in den Ansprüchen 1 bis 6 erwähnten Bedeutungen besitzt,

$R_4$' ein Wasserstoffatom darstellt oder die für $R_4$ eingangs erwähnten Bedeutungen besitzt und

X' eine Iminogruppe darstellt oder die für X in den Ansprüchen 1 bis 6 erwähnten Bedeutungen besitzt, wobei jedoch entweder $R_4$' ein Wasserstoffatom oder X' eine Iminogruppe darstellen muß, mit einer Verbindung der Formel

$$R_7 - CHO \qquad ,(V)$$

in der

$R_7$ ein Wasserstoffatom, eine Methyl- oder Äthylgruppe darstellt, in Gegenwart eines Reduktionsmittels umgesetzt wird oder

c) zur Herstellung von Verbindungen der Formel I, in der X eine Sulfinyl- oder Sulfonylgruppe darstellt, eine Verbindung der allgemeinen Formel

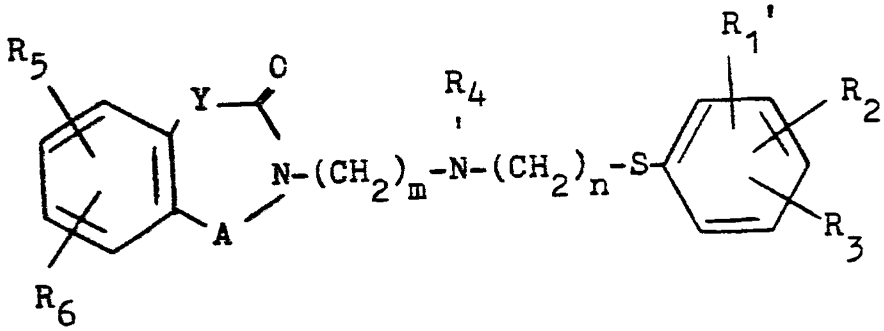

,(VI)

in der

A, $R_2$ bis $R_6$, Y, m und n wie in den Ansprüchen 1 bis 6 definiert sind und

$R_1$' eine durch eine Schutzgruppe geschützte Hydroxy-, Amino-, oder Alkylaminogruppe darstellt oder die für $R_1$ erwähnten Bedeutungen besitzt, oxidiert und gegebenenfalls anschließend ein verwendeter Schutzrest abgespalten wird oder

d) zur Herstellung von Verbindungen der allgemeinen Formel I, in der X eine gegebenenfalls durch eine Alkylgruppe substituierte Iminogruppe, ein Sauerstoff- oder Schwefelatom darstellt, eine Verbindung der allgemeinen Formel

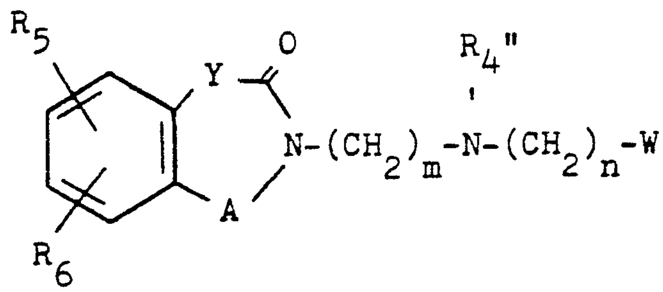

,(VII)

in der

A, $R_5$, $R_6$, Y, m und n wie in den Ansprüchen 1 bis 6 definiert sind,

$R_4$" eine Schutzgruppe für eine Aminogruppe oder mit Ausnahme des Wasserstoffatoms die für $R_4$ in den Ansprüchen 1 bis 6 erwähnten Bedeutungen besitzt und

W eine nukleophile Austrittsgruppe darstellt, mit einer Verbindung der allgemeinen Formel

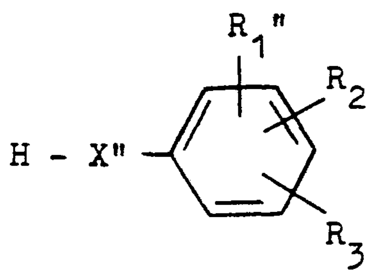

,(VIII)

in der

$R_2$ und $R_3$ wie in den Ansprüchen 1 bis 6 definiert sind,
$R_1$" eine durch einen Schutzrest geschützte Amino-, Alkylamino- oder Hydroxygruppe, ein Wasserstoff-, Chlor- oder Bromatom, eine Dialkylamino- oder Alkoxygruppe darstellt, wobei jeder Alkylteil 1 bis 3 Kohlenstoffatome enthalten kann, und
X" eine gegebenenfalls durch eine Alkylgruppe mit 1 bis 3 Kohlenstoffatomen substituierte Iminogruppe, ein Sauerstoff- oder Schwefelatom bedeuten, umgesetzt und gegebenenfalls anschließend ein verwendeter Schutzrest abgespalten wird oder

e) zur Herstellung von Verbindungen der allgemeinen Formel I, in der Y eine Carbonylgruppe darstellt, eine Verbindung der allgemeinen Formel

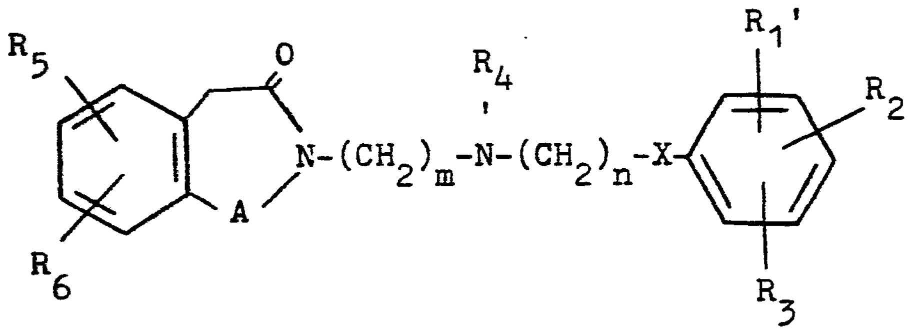

,(IX)

in der

A, $R_2$ bis $R_6$, X, m und n wie in den Ansprüchen 1 bis 6 definiert sind und

$R_1$' eine durch einen Schutzrest geschützte Hydroxy-, Amino- oder Alkylaminogruppe darstellt oder die für $R_1$ in den Ansprüchen 1 bis 6 erwähnten Bedeutungen besitzt, oxidiert und gegebenenfalls ein verwendeter Schutzrest abgespalten wird oder

f) zur Herstellung von Verbindungen der allgemeinen Formel I, in der Y eine gegebenenfalls durch eine Benzyl- oder Alkylgruppe substituierte Iminogruppe darstellt, eine Verbindung der allgemeinen Formel

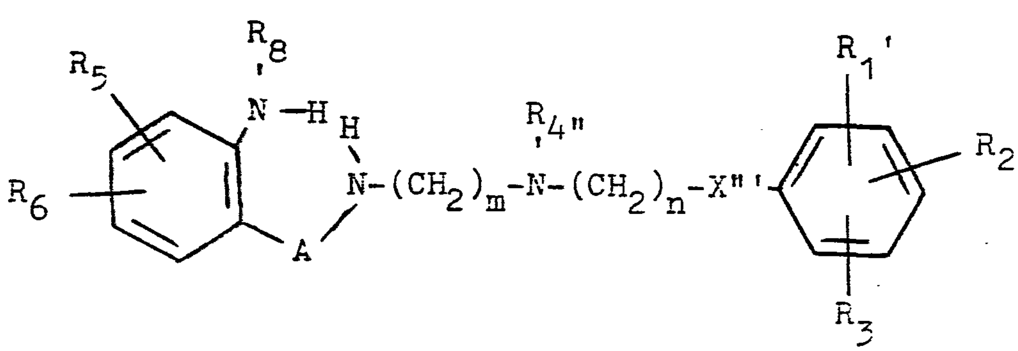

,(X)

in der
$R_2$, $R_3$, $R_5$, $R_6$, A, m und n wie in den Ansprüchen 1 bis 6 definiert sind,
$R_1$' eine durch einen Schutzrest geschützte Hydroxy-, Amino- oder Alkylaminogruppe darstellt oder die für $R_1$ eingangs erwähnten Bedeutungen besitzt,
$R_4$" eine Schutzgruppe für eine Iminogruppe oder mit Ausnahme des Wasserstoffatoms die für $R_4$ in den Ansprüchen 1 bis 6 erwähnten Bedeutungen besitzt,
X"' eine durch eine Schutzgruppe geschützte Iminogruppe oder mit Ausnahme der Iminogruppe die für X in den Ansprüchen 1 bis 6 erwähnten Bedeutungen besitzt und $R_8$ ein Wasserstoffatom, eine Benzyl- oder Alkylgruppe mit 1 bis 3 Kohlenstoffatomen bedeuten, mit einem Kohlensäurederivat der allgemeinen Formel

$$W - CO - W \qquad ,(XI)$$

in der
W, die gleich oder verschieden sein können, jeweils eine nukleophile Austrittsgruppe bedeuten, umgesetzt und gegebenenfalls anschließend ein verwendeter Schutzrest abgespalten wird oder

g) zur Herstellung von Verbindungen der allgemeinen Formel I, in der $R_1$ eine Alkoxy-, Amino-, Alkylamino- oder Dialkylaminogruppe und mindestens einer der Reste $R_2$ oder $R_3$ ein Chlor- oder Bromatom darstellen, eine Verbindung der allgemeinen Formel

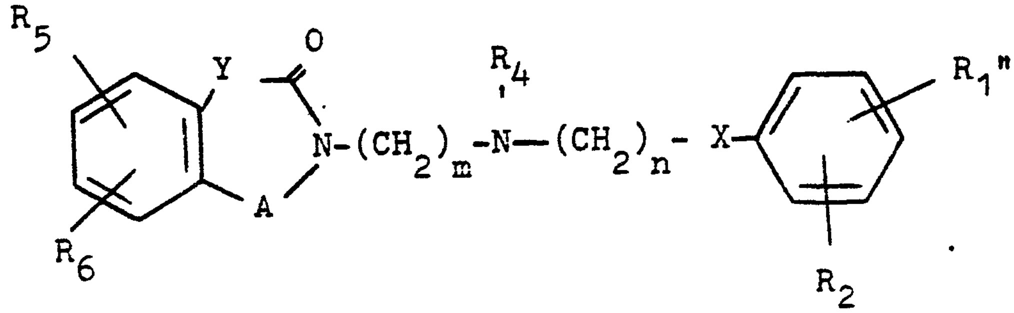

,(XII)

in der A, $R_2$, $R_4$ bis $R_6$, X, Y, m und n wie in den Ansprüchen 1 bis 6 definiert sind und $R_1$" eine Alkoxy-, Amino-, Alkylamino- oder Dialkylaminogruppe darstellt, halogeniert wird oder

h) zur Herstellung von Verbindungen der allgemeinen Formel I, in der $R_4$ ein Wasserstoffatom oder X eine Iminogruppe oder Y eine Iminogruppe oder $R_4$ ein Wasserstoffatom und X und/oder Y eine Iminogruppe darstellen, eine Verbindung der allgemeinen Formel

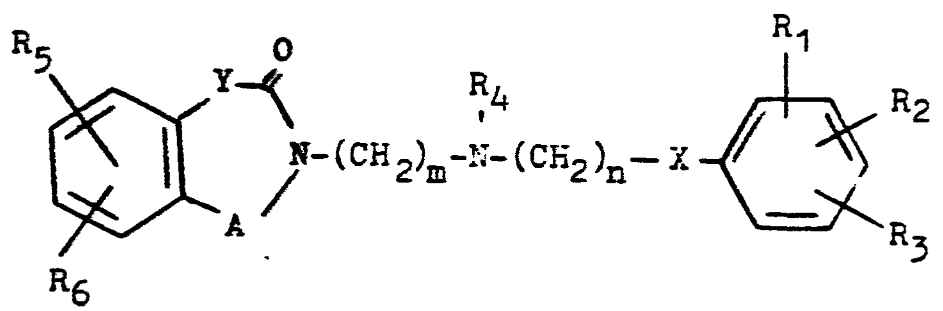

,(XIII)

in der $R_1$ bis $R_6$, A, X, Y, m und n wie in den Ansprüchen 1 bis 6 definiert sind, wobei jedoch $R_4$ eine Benzylgruppe oder X eine Benzyliminogruppe oder Y eine Benzyliminogruppe darstellen muß, entbenzyliert wird und

gewünschtenfalls anschließend eine so erhaltene Verbindung der allgemeinen Formel I in ihr Säureadditionssalz, insbesondere in ihr physiologisch verträgliches Säureadditionssalz, mit einer anorganischen oder organischen Säure übergeführt wird.